# FASCICULE DE BREVET EUROPEEN

(11) **EP 4 083 352 B1**
(45) Date de publication et mention de la délivrance du brevet: **11.09.2024**
(21) Numéro de dépôt: 22167413.8
(22) Date de dépôt: 23.01.2020
(51) Int. Cl.: E05B 1/00, A61L 2/18, E05B 47/00

(54) **DISPOSITIF DE NETTOYAGE AUTOMATIQUE D'UNE POIGNÉE**
VORRICHTUNG ZUR AUTOMATISCHEN REINIGUNG EINER KLINKE
DEVICE FOR AUTOMATIC CLEANING OF A HANDLE

(30) Priorité: 24.01.2019 EP 19153558
(43) Date de publication de la demande: 02.11.2022
(62) Demande divisionnaire de: 20701194.1
(73) Titulaire: Cleanmotion Sàrl, 1005 Lausanne (CH)
(72) Inventeur: HORVATH, Alex, 1005 Lausanne (CH); VAN SPROLANT, Valentin, 74380 Bonne (FR); BARILLA, Giovanni, 1206 Geneva (CH)
(74) Mandataire: reuteler & cie SA

(56) Documents cités:
- EP-A1- 0 351 307
- CH-A2- 699 581
- DE-A1- 102013 009 098
- GB-A- 2 433 878

## Description

La présente invention concerne un dispositif de nettoyage automatique d'une poignée par exemple une poignée d'ouverture d'un ouvrant telle qu'une porte, une fenêtre, porte-fenêtre ou une poignée d'appui ou encore une rambarde ou une rampe.

Dans certains lieux publics à forte fréquentation et/ou sensibles, comme les toilettes, les hôpitaux, les blocs opératoires, les hôtels, restaurants, les commerces, les bureaux, etc., il est important de maintenir une bonne hygiène sur les principales surfaces de contact pour éviter la transmission de maladies. Les poignées présentent des surfaces sur lesquelles se concentrent les bactéries, virus ou autres microorganismes. Un dépôt gras s'accumule aussi sur ces surfaces. Il est donc important et recommandé de les nettoyer régulièrement avec un produit nettoyant et de préférence, ayant des propriétés désinfectantes.

Ainsi, le nettoyage des surfaces comme les poignées est souvent réalisé de manière irrégulière et incomplète. Or la transmission de microorganismes (virus, bactéries, champignons, etc.) peut se faire facilement et rapidement par simple touché d'une surface infectée. Il est donc difficile d'éliminer le risque de transmission par les méthodes traditionnelles de nettoyage.

De nombreux systèmes de nettoyage automatique de poignées sont divulgués dans l'art antérieur, par exemple tels que décrits dans les publications de brevets/demandes de brevets US7360674, EP3118395, DE102010011309A1, US20100140499, US7989779, US20120176241, US20140137369, EP0351307, US20050011042, EP1164234, CH699581, et DE102013009098.

CH699581 divulgue un dispositif de nettoyage d'une poignée comprenant au moins poignée avec une surface de préhension à nettoyer faisant partie d'une paroi de forme tubulaire et un organe d'application pour appliquer un liquide nettoyant sur la surface de préhension, l'organe d'application formé en partie au moins d'une bague annulaire montée autour de la surface de préhension et comprenant une tampon agencée au contact de la surface de préhension, ladite tampon ayant une structure permettant de stocker le liquide. DE1 02013009098 divulgue un dispositif de nettoyage d'une poignée avec des moyens d'alimentation avec un réservoir qui permet le rechargement de la réserve tampon.

Les poignées peuvent être fortement sollicitées et leur omniprésence fait que les utilisateurs ne prêtent pas beaucoup d'attention ou de soin particulier lors de l'usage d'une poignée. Des poignées avec des mécanismes de nettoyage doivent rester compactes tout en étant fiables et robustes, requérant une maintenance minimale comme pour une poignée sans dispositif de nettoyage. L'autonomie et la faible consommation de produit de nettoyage sont également importantes. Ces diverses contraintes sont toutefois difficiles à réunir et aucun système existant ne permet d'atteindre tous les buts recherchés dans une configuration qui doit aussi être économique.

Il existe donc un besoin pour proposer une solution satisfaisante pour le nettoyage d'une poignée, dont les fonctionnalités et l'ergonomie sont conservées, qui soit efficace.

Un objet de l'invention est de fournir un dispositif de nettoyage d'une poignée compacte, performante, fiable et économe à installer et à utiliser.

Il est avantageux, pour certaines applications, de fournir un dispositif de nettoyage d'une poignée autonome dans son mode de fonctionnement d'un point de vue énergétique.

Des objets de l'invention sont réalisés par un dispositif de nettoyage d'une poignée selon la revendication indépendante.

Les revendications dépendantes décrivent des caractéristiques avantageuses de l'invention.

Dans la présente, on décrit un dispositif de nettoyage d'une poignée comprenant au moins une poignée comprenant une surface de préhension à nettoyer, faisant partie d'une paroi de forme tubulaire ou partiellement tubulaire, un organe d'application pour appliquer un liquide nettoyant sur la surface de préhension, et des moyens d'alimentation en liquide nettoyant comprenant au moins un réservoir pour recevoir et distribuer du liquide nettoyant à l'organe d'application. L'organe d'application comprend au moins une réserve tampon agencée au contact de la surface de préhension et en ce que le dispositif comprend une unité d'actionnement de l'organe d'application comprenant des moyens d'entraînement configurés pour déplacer l'organe d'application axialement le long de ladite surface de préhension entre une position de distribution, dans laquelle l'organe d'application est en vis-à-vis du collecteur de distribution, ou communication fluidique avec celui-ci, pour le transfert de liquide nettoyant dans la réserve tampon et une configuration d'application pour l'application de liquide nettoyant sur la surface de préhension de façon à appliquer du liquide nettoyant depuis la réserve tampon sur la surface de préhension lors de ce déplacement relatif.

Un aspect de l'invention se base sur le principe de la mobilité relative d'un organe de nettoyage sur la surface contaminée et contenant un volume de liquide réduit, de préférence prédosé en fonction de la surface à nettoyer. Ainsi, le dispositif est efficace, peu encombrant et ses besoins en énergie pour le faire fonctionner sont faibles. La réserve tampon est préférablement configurée de façon à contenir un volume de liquide nettoyant déterminé pour nettoyer la totalité de la surface de préhension lors du déplacement relatif. De ce fait, le nettoyage est complet et reproductible à chaque cycle. Le rechargement de la réserve tampon par l'activation des moyens d'alimentation en liquide peut aussi se faire en temps masqué, et non pas au moment du nettoyage lors du déplacement relatif de l'organe d'application contre la surface à traiter. Il est ainsi plus facile de contrôler la dose de liquide nécessaire au nettoyage de la surface. Il en résulte un moindre risque de défaut d'alimentation en liquide lors de l'étape de nettoyage. Enfin, cela rend possible par des moyens de collecte (décrits plus loin) l'utilisation de l'énergie produite par l'actionnement mécanique de la poignée comme lors de l'ouverture de l'ouvrant.

La surface de préhension n'est pas nécessairement tubulaire et peut être partiellement tubulaire au sens où elle peut former une surface en partie ouverte longitudinalement comme une surface en forme transversale hélicoïdale, en U ou en C ou autre.

Selon une autre configuration possible, la surface de préhension pourrait être par exemple formée d'une série de plusieurs tubes de petits diamètres arrangés individuellement en rotation libre selon leur propre axe individuel et arrangés ensemble dans une disposition circulaire autour d'un axe médian longitudinal de la poignée formant ainsi un ensemble tubulaire ou semi-tubulaire de plus grand diamètre.

Le terme « nettoyage » se rapporte essentiellement à une opération de désinfection (c'est-à-dire de destruction de microorganismes ou germes) et possiblement de dégraissage. Le terme « liquide nettoyant » se rapporte essentiellement à un liquide ayant des propriétés désinfectantes et possiblement dégraissantes. De préférence le liquide est constituée ou contient une solution hydro-alcoolique et/ou une solution contenant des agents tensio-actifs ou savonneux capable d'éliminer ou dissoudre les corps gras.

De préférence, les moyens d'alimentation en liquide comprennent une pompe qui peut être actionnée de manière contrôlée électroniquement ou mécaniquement par actionnement de la poignée. La pompe peut être une pompe péristaltique ou une pompe à piston ou au d'un autre type. Dans un arrangement possible, la pompe est configurée pour alimenter la réserve tampon sous l'effet d'une action mécanique appliquée sur la pompe par au moins un élément d'actionnement de la poignée associé au mouvement de la poignée en rotation lors de l'actionnement d'un mécanisme d'ouverture et/ou de fermeture de l'ouvrant.

Selon l'invention, la réserve tampon comprend un tampon applicateur en contact avec la surface de préhension ayant une structure permettant de stocker le liquide dans une structure poreuse ou d'interstices de rétention du liquide tel que par effet de capillarité. Une telle configuration de la réserve tampon a pour avantage de pouvoir mieux réguler l'application et l'étalement du liquide sur la surface, tout en minimisant avantageusement le risque de fuites. Il a aussi pour avantage de mettre à disposition un volume de liquide dimensionné pour couvrir toute la surface de préhension lors d'un seul cycle de déplacement relatif (surface de préhension/organe d'application) lors du nettoyage.

Le matériau du tampon applicateur peut être avantageusement compressible. La compressibilité assure aussi une meilleure régularité de l'application et réduit le risque de spots non-couverts par le liquide. Le matériau peut être : une éponge ou une mousse, une brosse de fils, une épaisseur ou un enroulement de fibres, une structure en nid d'abeille, des billes en polymère ou tout matériau muni d'une multitude de cavités ou interstices de faibles volumes formant un réseau de régulation du flux de sortie et/ou d'étalement du liquide. La capacité en liquide de la réserve tampon dépend de la surface de préhension à traiter mais peut être par exemple comprise entre 0.05 à 3 ml, de préférence entre 0.1 et 1 ml.

La dimension transversale du tampon applicateur peut être prévue pour que le tampon applicateur couvre toute la surface à traiter lors du déplacement relatif. Le nettoyage peut ainsi être obtenu selon les cas par un déplacement unidirectionnel ou d'un seul passage ou par un déplacement bidirectionnel ou selon un aller-retour de l'organe d'application sur la surface de préhension ou inversement. Dans tous les cas, le déplacement est de préférence le plus court possible et ne nécessite pas de mouvements ou de trajets ou trajectoires complexes.

Selon l'invention, le dispositif comprend un collecteur de distribution de liquide nettoyant comprenant au moins une entrée de liquide en communication avec le réservoir de liquide et une pluralité de sorties de liquide distribuée en regard du tampon applicateur de l'organe d'application. Ainsi, un arrangement avec plusieurs sorties de liquide permet de distribuer mieux et plus rapidement le liquide au travers du tampon applicateur notamment sur toute sa largeur. Cet arrangement augmente l'efficacité de recouvrement de la surface traitée, évite les zones surchargées en liquide et réduit aussi le volume total de liquide nécessaire pour un cycle de nettoyage.

Selon l'invention, l'organe d'application est en partie au moins une bague annulaire montée autour de la surface de préhension, agencée pour se déplacer axialement le long de ladite surface entre une position de distribution dans laquelle l'organe d'application est en vis-à-vis du collecteur de distribution, ou communication fluidique avec celui-ci, pour le transfert de liquide nettoyant dans la réserve tampon et une configuration d'application pour l'application de liquide nettoyant sur la surface de préhension. De préférence, l'organe d'application comprend un support annulaire externe rigide à l'intérieur duquel est monté de manière coaxiale un tampon applicateur en forme d'anneau ayant une surface d'application en contact avec la surface de préhension. Dans ce mode de réalisation, le dispositif reste ergonomique et l'organe d'application du dispositif est moins encombrant puisqu'il peut être placé sur un côté de la poignée donc sans gêne lorsque celle-ci est opérée dans sa fonction de préhension.

Dans ce cas, la chambre de distribution de liquide de l'organe d'application comprend préférentiellement une série de sorties de liquide distribuées sur au moins une circonférence de la surface de préhension dans une zone de transfert ou de rechargement ; lesdites sorties étant agencées en regard d'une portion annulaire du tampon applicateur en position de rechargement ou d'alimentation distribution.

Dans le cas d'un déplacement axial de l'organe d'application vis-à-vis de la surface de préhension qui reste statique sur l'embase de la poignée, le dispositif est simplifié. Le tampon applicateur est séparable du collecteur de de distribution en liquide qui peut faire partie de la paroi de la surface de préhension et/ou de l'embase de la poignée; ce qui permet de simplifier l'organe d'application et d'éviter les problèmes de connexion fluidique associés au raccordement de pièces mobiles. Le collecteur de distribution statique fait ainsi office de station de recharge en liquide de position définie fixe pour le tampon applicateur ; lequel peut se mettre en position de repos et de rechargement ou d'alimentation en vis-à-vis du collecteur avant et/ou après un cycle de nettoyage.

En particulier, l'organe d'application du tampon applicateur peut être déplacé axialement le long de la surface de préhension au moyen d'un moyen d'entrainement à vis, à courroie, un engrenage sans contact du type électromécanique. A titre d'exemple, ce moyen d'entraînement comprend une vis sans fin s'étendant longitudinalement à l'intérieur de paroi tubulaire de la surface de préhension de la poignée et un écrou solidaire déplaçable le long de la vis sans fin et solidaire de l'organe d'application par une liaison traversant une ouverture ou une rainure longitudinale au travers de la paroi de la surface de préhension ou avec un couplage magnétique de l'anneau de l'organe d'application avec l'écrou de la vis sans fin (donc sans ouverture ou rainure nécessaire) permettant ainsi le déplacement axial de l'organe d'application lors de l'entraînement de la vis sans fin en rotation.

Dans un autre mode de réalisation, dans le cas d'un couplage magnétique, l'anneau de l'organe d'application peut être entrainé par un moteur linéaire capable de créer un champ électromagnétique de déplacement de l'organe le long de la surface de préhension.

Le contrôle des moyens d'entraînement et/ou des moyens d'alimentation peut se faire au moyen d'un moteur électrique et/ou de manière mécanique. L'énergie électrique et/ou mécanique peut avantageusement être une énergie collectée, au moins partiellement, par la force motrice appliquée par l'utilisateur sur la poignée lors de l'actionnement de la poignée sur un mécanisme d'ouverture et/ou de fermeture de l'ouvrant commandée par la poignée. Des modes préférentiels seront décrits plus loin.

Selon un mode de contrôle possible, le dispositif selon l'invention est commandé au moyen d'un contrôleur électronique telle qu'une unité centrale pouvant être logée dans la poignée ou en dehors comme dans un boitier, une partie de l'ouvrant ou un encadrement. Ainsi, l'unité d'actionnement comprend un contrôleur électronique configuré pour commander automatiquement l'action des moyens d'entraînement pour le déplacement relatif de la réserve tampon avec la surface de préhension et/ou commander la distribution en liquide par les moyens d'alimentation pour remplir en liquide la réserve tampon de l'organe d'application à partir du réservoir de liquide. Plus particulièrement, le contrôleur électronique commande l'actionnement d'un moteur électrique du moyen d'entraînement. Le contrôleur peut contrôler séquentiellement ou concomitamment l'alimentation en liquide par actionnement de la pompe et l'activation des moyens d'entraînement notamment le moteur électrique.

Selon un mode avantageux, le dispositif comprend au moins un capteur agencé pour détecter la présence du touché de la poignée par un utilisateur, ledit capteur étant associé au contrôleur électronique lequel est configuré pour actionner automatiquement les moyens d'alimentation du liquide et/ou d'entraînement, en réponse à une information de détection. La présence d'au moins un capteur permet ainsi d'activer la séquence de nettoyage selon un mode de détection pouvant être préétabli par programmation du contrôleur par exemple juste après la détection de la présence d'une main sur la poignée par le (les) capteur(s), ou avant un contact d'une main sur la poignée, par la détection d'un mouvement de commande de l'utilisateur. Par exemple, après détection de présence par le (les) capteur(s), le contrôleur est programmé pour activer les moyens d'entraînement pour effectuer l'application du liquide sur la surface de préhension.

Le capteur peut être un capteur de présence ou d'effort à effet capacitif, optique, piézoélectrique, piézo-résistif ou à ultrasons. Par exemple, un ou plusieurs capteurs sont positionnés sur ou à l'intérieur de la surface de préhension de la poignée notamment dans les zones les plus exposées comme au centre et/ou à une extrémité libre de la surface de préhension ou de l'embase.

Dans un mode d'alimentation possible utilisant une pompe électrique contrôlée, la pompe peut être activée suite à la détection par le/les capteur(s) pendant un temps prédéterminé correspondant au volume de remplissage de la réserve tampon. Le lancement de l'étape d'alimentation peut être éventuellement initiée à tout moment comme dès la détection du touché ou après la détection du touché et/ou après un temps prédéterminé. L'étape d'application ne peut être lancée quant à elle qu'après détection et une fois que le capteur ne détecte plus le touché ou la présence sur la surface ou l'embase.

Dans un autre mode de réalisation, le dispositif comprend une pompe actionnée par un moyen d'actionnement mécanique qui transmet un effort résultant d'un mouvement de pivot de la poignée actionnée par l'utilisateur, par exemple l'abaissement et/ou le retour horizontal de la poignée, comme lors de l'ouverture et/ou fermeture de l'ouvrant. La pompe peut être une pompe du type péristaltique ou à piston par exemple. Le moyen d'actionnement mécanique peut comprendre au moins une rampe ou un moyen de came lequel agit sur un moyen de compression péristaltique ou un piston de la pompe sous l'effet du mouvement de rotation de la poignée actionnée par la force motrice de l'utilisateur comme son abaissement et/ou sa relevée.

Selon un aspect avantageux de l'invention, le dispositif est configuré pour être autonome en énergie. De préférence, de l'énergie mécanique provenant de l'actionnement de la poignée est utilisée et stockée sous forme d'énergie électrique, mécanique ou une combinaison de ces deux types d'énergie pour être ensuite utilisée pour actionner la pompe et/ou les moyens d'actionnement pour le déplacement relatif de l'organe d'application et/ou de la surface de préhension.

Ainsi, le dispositif comprend au moins un bloc de stockage et d'inversion ou un organe de récupération d'énergie mécanique par des moyens de transmission de couple du fait de la rotation de la poignée sur un mécanisme d'ouverture et/ou fermeture d'un ouvrant (porte, fenêtre, etc.), pour le stockage de cette énergie mécanique et/ou de transformation et stockage en énergie électrique et enfin de redistribution de cette énergie mécanique ou électrique stockée à l'unité d'actionnement ou aux moyens d'entraînement ou à un ensemble formé d'un bloc de translation et un bloc de couplage qui commande le déplacement de la surface de préhension ou de l'organe d'application.

Selon un premier mode possible, l'organe de récupération d'énergie est du type électrique. En particulier, l'organe de récupération d'énergie peut comprendre un générateur de conversion d'énergie mécanique en énergie électrique tel qu'une dynamo, avec ou sans multiplicateur de vitesse, associée aux moyens de transmission de couple et comprend un accumulateur d'énergie électrique tel qu'au moins un supercondensateur et/ou une batterie configurée pour stocker l'énergie électrique ainsi générée par le générateur et la mettre à disposition pour alimenter les moyens d'entraînement et/ou le contrôleur électronique.

Selon un mode de réalisation préféré, l'organe de récupération et de restitution d'énergie est du type mécanique.

En particulier, l'organe de récupération est un organe d'accumulation d'énergie mécanique, de restitution et de transmission d'énergie mécanique ou bloc de stockage et d'inversion comprenant au moins un mécanisme de transmission avec au moins un arbre pour transmettre un couple à un mécanisme de fermeture d'ouvrant, au moins un premier ressort agencé pour emmagasiner l'énergie mécanique lors du déplacement dudit arbre, un premier embrayage unidirectionnel et un ensemble de loquets cycliques actionnés par un arbre à came pour restituer l'énergie emmagasinée par le premier ressort à des moyens de transmission et de couplage, ou bloc de translation et bloc de couplage, configurés pour déplacer l'organe d'application relativement à la surface de préhension dans une première direction.

Selon un exemple préférentiel, l'organe d'application est déplacé sur la surface de préhension depuis une position d'alimentation à une position de fin de course. Dans ce cas, l'organe d'application de préférence de forme annulaire se déplace de préférence axialement sur la surface de préhension comme précédemment mentionné. Selon un autre exemple, c'est la surface de préhension, par exemple de forme hélicoïdale (comme décrit dans un mode de la présente demande) qui est déplacée en rotation vis-à-vis d'un organe d'application en forme de barre nettoyante.

Selon un exemple, les moyens de transmission et de couplage peuvent comprendre un bloc de translation et un bloc de couplage pour déplacer l'organe d'application en translation sur la surface de préhension. Le bloc de translation peut comprendre un ensemble formé par une vis sans fin et un écrou par exemple ou un ensemble de courroie et poulies associé à un élément de connexion associé au bloc de couplage.

Le bloc de couplage peut former un couplage direct par tige ou équivalent avec l'organe d'application. Alternativement, le bloc de couplage peut être un organe de couplage magnétique permettant de transmettre l'effort du bloc de translation à l'organe d'application sans liaison mécanique.

De plus, l'organe d'accumulation d'énergie mécanique, de restitution et de transmission d'énergie mécanique ou bloc de stockage et d'inversion peut comprendre des moyens d'inversion de mouvement configurés pour déplacer l'organe d'application relativement à la surface de préhension dans une seconde direction opposée à la première direction.

Selon un exemple préférentiel, ces moyens d'inversion comprennent un second embrayage cyclique et un second ressort configurés pour déplacer l'organe d'application dans la seconde direction, par exemple pour rappeler l'organe d'application de sa position de fin de course à sa position d'alimentation, ledit second embrayage étant arrangé entre le second ressort et le premier ressort afin d'entraîner librement l'organe d'application sans contrainte du premier ressort.

Selon un aspect de l'invention, les moyens d'alimentation en liquide comprennent au moins une pompe actionnée de manière contrôlée électroniquement par une unité électronique ou mécaniquement par des moyens d'actionnement mécanique lors de l'actionnement de la poignée de préférence le pivotement de celle-ci autour de son axe d'actionnement pour l'actionnement d'un mécanisme d'ouverture/fermeture de l'ouvrant. De préférence, la pompe est une pompe péristaltique ou une pompe à piston.

Selon un aspect possible de l'invention, le réservoir de liquide peut être contenu dans un conteneur amovible connectée par des moyens de connexion détachables à au moins une interface de connexion des moyens d'alimentation. L'interface de connexion peut être contenue dans le châssis de la poignée ou dans un boitier solidaire de l'ouvrant. L'interface peut être munie de moyens de connexions complémentaires et communiquant avec l'organe d'application via au moins un conduit.

Selon un mode de réalisation avantageux, le réservoir des moyens d'alimentation en liquide est situé au-dessus de la poignée, l'apport d'un volume de liquide dispensé à la réserve tampon se faisant par l'ouverture d'une vanne actionnée mécaniquement ou électriquement pour laisser le liquide passer uniquement par la pression due à la force gravitationnelle.

Selon un mode de réalisation avantageux, la réserve tampon comprend un tampon applicateur en contact avec la surface de préhension monté de manière démontable dans un support rigide de la bague afin de pouvoir remplacer ou nettoyer le tampon applicateur.

Selon un mode de réalisation avantageux, le tampon applicateur est sous forme d'un anneau fendu flexible de sorte à pouvoir passer la poignée à travers une fente ouverte de l'anneau fendu.

Selon un mode de réalisation avantageux, le support rigide de la bague comprend au moins deux parties séparables, les deux parties verrouillées ensemble par un mécanisme de fixation élastique ou à aimant, et déverrouillable avec un outil comprenant une goupille.

Selon un mode de réalisation avantageux, le support rigide de la bague comprend au moins deux parties séparables, les deux parties verrouillées ensemble par un mécanisme de fixation magnétique.

Selon un mode de réalisation avantageux, les deux parties du support rigide de la bague, comprennent une base et un couvercle, le couvercle étant logé dans la base sous un recouvrement annulaire.

Selon un mode de réalisation, le dispositif comprend en outre un outil de déverrouillage comprenant un aimant produisant un champ magnétique plus fort que le champ magnétique du mécanisme de fixation.

Selon un mode de réalisation avantageux, la bague annulaire de l'organe d'application comprend au moins un aimant permanent logé dans un support rigide de la bague, le support rigide comprenant ou constitué d'un matériau ferromagnétique.

Selon un mode de réalisation avantageux, la surface de préhension comprend une rainure circulaire intersectant des trous de distribution des moyens d'alimentation pour l'irrigation d'une zone tampon correspondant à une position initiale de la réserve tampon.

D'autres caractéristiques et avantages de l'invention ressortiront à la lecture de la description qui suit, en référence aux figures annexées, qui illustrent :
La figure 1 est une vue de face schématique générale du dispositif de nettoyage de la poignée installé sur une porte selon un mode de réalisation de l'invention;
- La figure 2 est un ordinogramme d'un exemple de méthode de nettoyage de la poignée selon le mode du dispositif de la figure 1;
- La figure 3 est une illustration schématique d'un organe de récupération d'énergie électrique du dispositif ;
- La figure 4 est une vue en perspective et par transparence d'une partie de la poignée comprenant une partie de l'organe de récupération d'énergie de la figure 3 ;
- La figure 5 est une vue en perspective partielle montrant un détail de l'organe de récupération d'énergie de la figure 4, en particulier, les moyens de transmission du couple par la poignée à l'ouvrant ;
- La figure 6 est une vue en perspective partielle montre un autre détail l'organe de récupération d'énergie de la figure 4 en particulier la liaison cinématique entre le moteur et les moyens de transmission du couple ;
- La figure 7 montre un élément des moyens de transmission du couple du dispositif des figures 4 à 6 ;

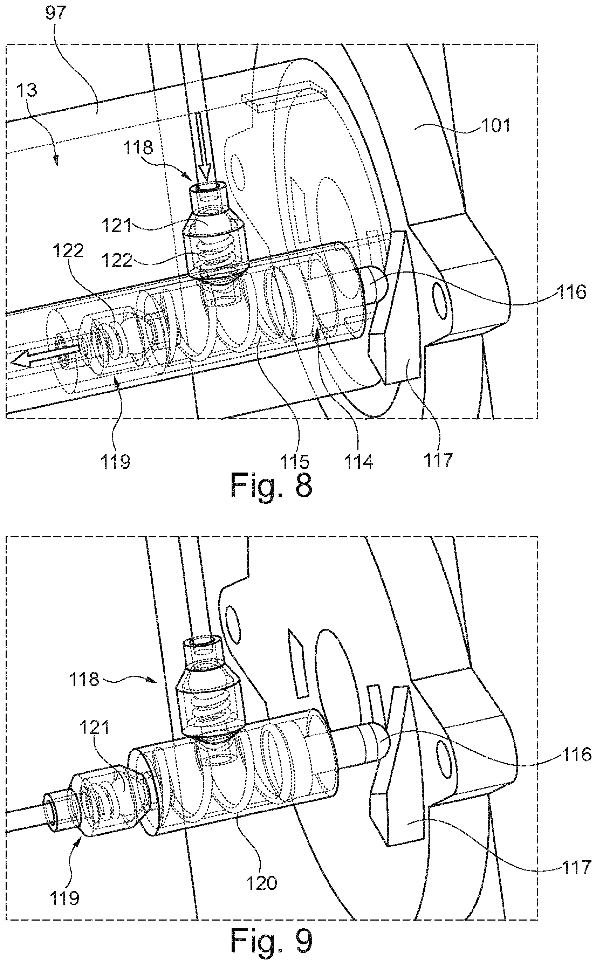

- La figure 8 montre un détail des moyens d'alimentation en liquide de la poignée en particulier une pompe à piston actionnées pas le mouvement de la poignée lors de l'actionnement d'un mécanisme d'ouverture et/ou fermeture de l'ouvrant ;
- La figure 9 montre un détail des moyens d'alimentation en liquide de la poignée sans le châssis de la poignée ;
- La figure 10 est une vue générale en perspective d'un dispositif de nettoyage de la poignée installé sur un ouvrant selon un mode de réalisation de l'invention dans une configuration d'alimentation en liquide ou de repos;
- La figure 11 est une vue générale en perspective du mode de réalisation de la figure 10 dans une configuration de nettoyage;
- La figure 12 est une vue en coupe selon un plan horizontal médian du dispositif de nettoyage selon un mode de réalisation;
- La figure 13 est une vue en perspective sans son habillage externe complet en particulier le recouvrement de la poignée par la paroi tubulaire de préhension du mode de réalisation de la figure 12;
- La figure 14 est une vue en perspective d'un dispositif de nettoyage de la poignée selon un mode de réalisation de l'invention dans une configuration en cours de nettoyage;
- La figure 14a est une vue agrandie de la partie « A » de la figure 14 ;
- La figure 15 est une vue en perspective de dos du dispositif de la figure 14 ;
- La figure 16 est une vue en section d'une partie du dispositif de la figure 15 ;
- La figure 17 est une vue en perspective d'une partie d'un dispositif de nettoyage de la poignée, avec un tube de préhension enlevé pour montrer l'intérieur, selon un mode de réalisation de l'invention ;
- La figure 18 est une vue en coupe d'une poignée d'un dispositif de nettoyage de la poignée selon un mode de réalisation de l'invention ;
- La figure 18a est une vue agrandie d'une partie de la figure 18 ;
- La figure 19 est une vue en coupe d'une partie d'une poignée d'un dispositif de nettoyage de la poignée selon un mode de réalisation de l'invention, en position de repos ;
- La figure 20 est une vue éclatée en perspective d'un organe d'application de liquide d'un dispositif de nettoyage de la poignée selon un mode de réalisation de l'invention ;
- La figure 21 est une vue en coupe d'un organe d'application de liquide d'un dispositif de nettoyage de la poignée et d'un outil d'ouverture de l'organe, selon un mode de réalisation de l'invention;
- La figure 22 est une vue élargie d'un détail du dispositif de la figure 12 en particulier du mécanisme d'entraînement, de récupération et de restitution d'énergie du dispositif (« Organe d'entraînement et d'énergie »);
- La figure 23 est une autre vue en perspective externe du dispositif de la figure 12 montrant l'organe d'entraînement, de récupération et de restitution de l'énergie mécanique du dispositif ;
- La figure 24 est une vue selon un autre angle de de l'organe d'énergie et d'entrainement du dispositif de la figure 12 ;
- La figure 25 est une vue sans le bâti selon un autre angle de l'organe d'énergie et d'entraînement de la poignée ;
- La figure 26 est une vue élargie de détail de l'organe de la figure 24 ;
- Les figures 27a à 27d sont des vues d'un détail de l'embrayage cyclique de l'organe de la figure 24, en positions couplée (Figs. 27a, 27b), respectivement découplée (Figs. 27c, 27d);
- La figure 28 est une illustration schématique et cinématique de différents modes de réalisation possibles du dispositif en fonction de différents modules sélectionnés de l'organe d'accumulation et de restitution d'énergie mécanique et des moyens de transmission et de couplage.

Faisant référence aux figures, un dispositif de nettoyage 1 comprend une poignée 2 avec une surface de préhension 3 qui peut former la surface d'une paroi de forme tubulaire ou partiellement tubulaire et qui s'étend le long d'un axe principal longitudinal médian I.

Une seconde poignée identique à la poignée 2 peut être prévue du côté opposé à l'ouvrant 9. Une seconde poignée standard peut être prévue de l'autre côté de l'ouvrant 9. Il est à noter que selon un mode possible, la poignée peut être montée de manière fixe, sans liaison avec un mécanisme d'ouverture, sur un ouvrant de type porte, fenêtre ou encore à un panneau, mur, sol ou tout autre support adéquat.

Le dispositif comprend aussi un organe d'application 10 configuré pour appliquer un liquide nettoyant sur la surface de préhension 3 et un réservoir 11 de liquide, contenant une solution de nettoyage, de capacité telle qu'elle permette une multitude de cycles de nettoyage. Des moyens d'alimentation 12 sont prévus pour alimenter l'organe d'application 10 en liquide en provenance du réservoir. Ces moyens 12 peuvent comprendre une pompe à liquide 13 ainsi qu'une conduite 14 reliant le réservoir à l'organe d'application 10.

Une méthode alternative pour irriguer le tampon applicateur 25 en désinfectant peut s'effectuer par gravité, utilisant un réservoir 11 situé au-dessus de la poignée 2 de porte. Le contrôle du volume de liquide dispensé se fait par l'ouverture/fermeture d'une vanne actionnée mécaniquement ou électriquement. Par exemple, lorsque la poignée est en position de repos, la vanne est fermée, et lorsque la poignée de porte est enclenchée, un mécanisme ouvre la vanne pour laisser le liquide passer uniquement par la pression due à la force gravitationnelle.

Le dispositif peut comprendre au moins un capteur 15 sur la surface 3 pour la détection de la présence d'une main ou du touché sur la surface. Le capteur 15 peut être un capteur de présence par exemple de type capacitif ou un capteur optique tel qu'un capteur infrarouge, ou un capteur à ultrasons. Le capteur peut être aussi un capteur de force ou de pression tel qu'une jauge de contrainte ou un capteur piézoélectrique. Le capteur peut aussi être un capteur de mouvement ou de force placé sur la poignée pour détecter un mouvement de la poignée sur son axe de pivot comme son abaissement lors de l'ouverture de l'ouvrant.

Le capteur 15 peut être configuré pour une détection de mouvement, par exemple un balayement de la main de l'utilisateur, afin d'enclencher un cycle de désinfection. Cette fonctionnalité permet d'offrir à l'utilisateur la possibilité d'avoir une poignée qui se désinfecte avant que celui-ci ne la touche pour avoir la confirmation visuelle que la désinfection a bien eu lieu.

D'autres capteurs peuvent être prévus comme encore un capteur de niveau dans le réservoir.

D'une manière générale, l'organe d'application 10 est commandé par une unité d'actionnement 16 comprenant un contrôleur électronique 17 et des moyens d'entraînement 18 de l'organe d'application 10. Le contrôleur 17 peut être logé dans la poignée elle-même comme dans l'embase 4 ou en dehors comme dans un boîtier intégré ou associé au support 9. Le contrôleur peut être également présent dans le boitier du réservoir 11.

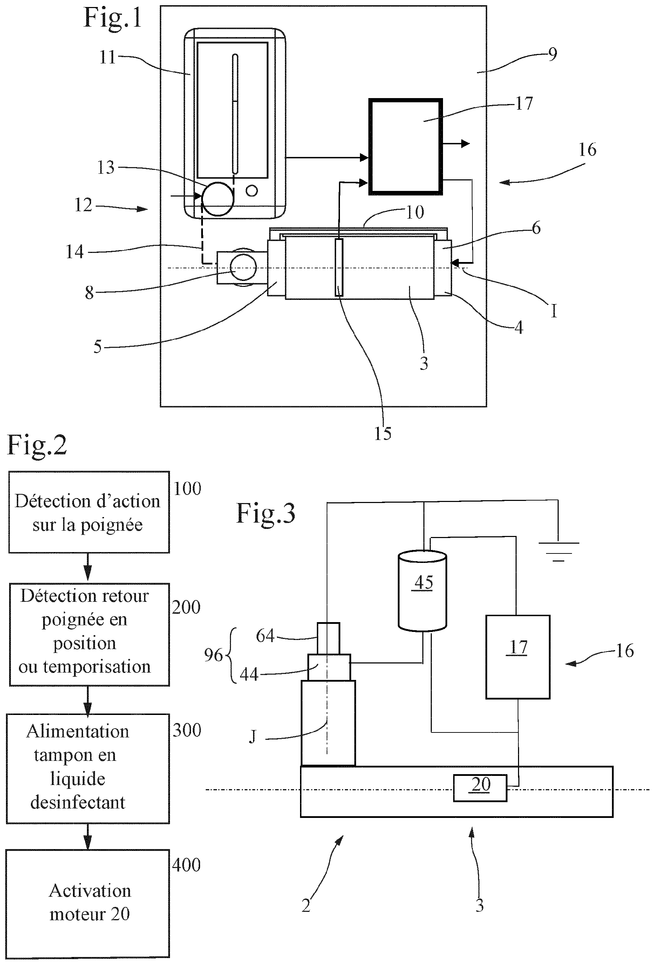

Un exemple de fonctionnement du dispositif selon un mode possible peut être expliqué en relation avec la figure 2. Dans une première étape 100, une action sur la poignée est détectée par le ou les capteurs de présence 15. Cette information est traitée par le contrôleur électronique 17. Cette étape peut être suivie d'une seconde étape de détection de l'arrêt de l'action sur la poignée 200 ou la poignée à l'état de repos. Pour cela, le contrôleur 17 reçoit l'information du capteur sur l'absence de présence. Dans ce cas, le contrôleur initie l'étape suivante 300 consistant à alimenter la réserve tampon 24 en liquide nettoyant par activation de la pompe 13. Selon un mode alternatif possible, la seconde étape 200 consiste à appliquer une temporisation suivant l'étape de détection 100 avant d'initier l'étape suivante 300. Selon un mode alternatif possible, l'actionnement de la pompe et/ou de l'organe d'application peut se faire par des moyens d'actionnement mécanique par l'action mécanique de déplacement de la poignée, par rotation ou autre mouvement, pour commander l'ouverture et/ou la fermeture de l'ouvrant. Une étape optionnelle de vérification du niveau de liquide peut être mise en oeuvre afin de vérifier la capacité du réservoir à remplir la réserve tampon. Si le niveau de liquide est insuffisant, la méthode peut être interrompue et un signal de rechargement émis par le contrôleur 17. Au cours de l'étape suivante 400, le contrôleur active les moyens d'entraînement 18, en particulier le moteur électrique 20, pour entraîner l'organe d'application. La translation a pour effet d'appliquer le liquide du tampon applicateur et de répartir son volume sur la surface de préhension. La couche de liquide peut être calibrée par un moyen de calibrage de préférence par une restriction comme une fente calibrée et/ou une lèvre transversale disposée entre la réserve tampon et la surface (non illustrée). Une fois la translation achevée, le contrôleur 17 commande l'arrêt des moyens d'entraînement 18. Le déplacement peut être contrôlé par tout moyen comme par un encodeur, par une mesure électrique comme la consommation de puissance du moteur ou encore par une temporisation. Il faut noter aussi que l'étape d'alimentation peut être mise en oeuvre entre les deux premières étapes de détection 100, 200 ou après la dernière étape d'actionnement 400.

La figure 3 illustre un principe général de récupération d'énergie électrique par un organe de récupération d'énergie 96 pour alimenter les composants électriques de la poignée notamment l'unité d'actionnement 16. Ainsi, le dispositif comprend un générateur de conversion d'énergie mécanique en énergie électrique 44. Le générateur est relié à un mécanisme de transmission de couple 64 relié au mécanisme de fermeture de l'ouvrant lequel transmet au générateur l'énergie mécanique lorsque la poignée est actionnée manuellement en pivot autour d'un axe d'actionnement J. Le générateur 44 peut être un moteur, un motoréducteur ou une dynamo. Le dispositif comprend aussi au moins un accumulateur d'énergie électrique 45 reliée électriquement au générateur pour stocker l'énergie électrique ainsi produite. Un tel accumulateur peut un (super)condensateur et/ou une batterie rechargeable configurée pour stocker l'énergie électrique ainsi générée. L'accumulateur est relié électriquement à l'unité d'actionnement 16 pour alimenter les moyens d'entraînement et le contrôleur électronique 17.

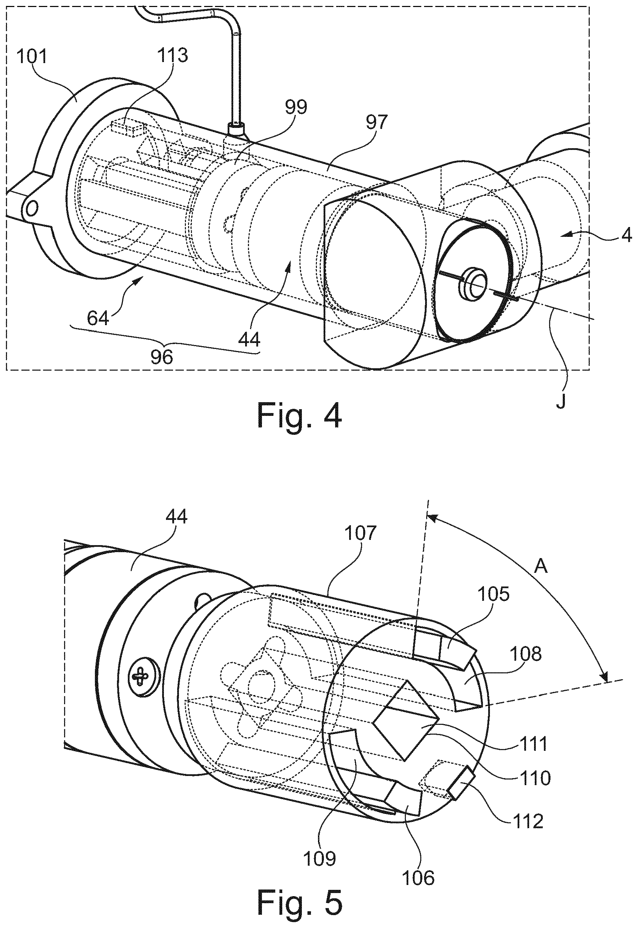

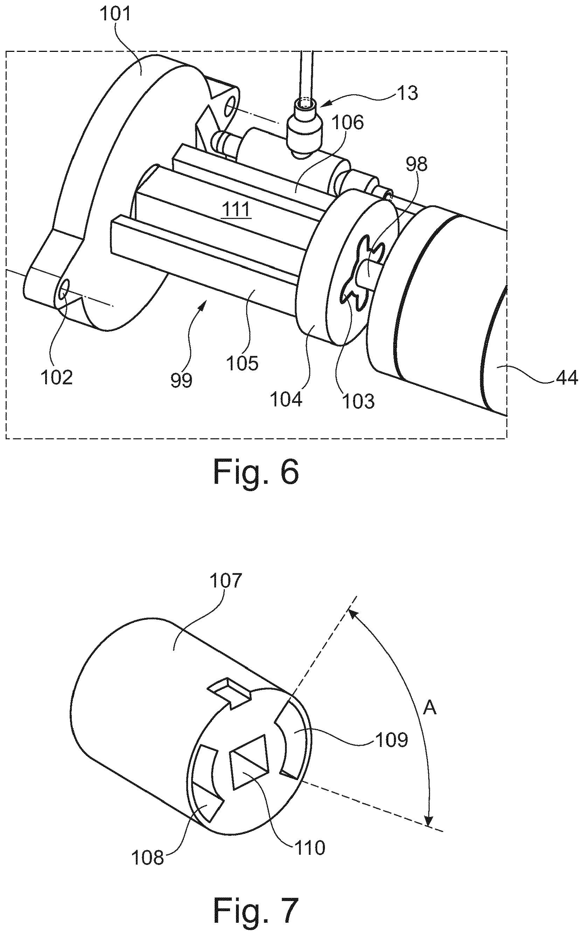

Les figures 4 à 7 illustrent un premier exemple d'organe de récupération d'énergie 96 fonctionnant sur la base du principe général de la figure 3. Ainsi, l'organe comprend un générateur électrique 44, tel qu'une dynamo, logé à l'intérieur d'un châssis tubulaire 97 de la poignée lequel est connecté à l'embase de préhension 4 de manière rigide pour former un coude par exemple. Le moteur électrique 44 est inséré dans le châssis de manière fixe selon l'axe longitudinal J du châssis correspondant à l'axe de pivotement de la poignée et comprend un arbre de transmission 98 lequel est solidaire d'une embase 99 configurée pour être fixé sur une paroi de l'ouvrant (non représenté). L'embase peut être fixée à l'ouvrant plus particulièrement par un flasque 101 de l'embase 99. Le flasque 101 peut avoir la forme d'un disque ou autre muni de trous 102 permettant la fixation à l'ouvrant au moyen de vis, rivets ou autres. L'arbre 98 du moteur peut être connecté par exemple par une pièce de raccordement 103 à une extrémité proximale 104 de l'embase 99. L'embase comprend des barres de transmission, par exemple deux barres parallèles 105, 106, qui relient l'extrémité proximale 104 à l'extrémité distale formée par le flasque 101. L'organe de transmission du couple comprend de plus un cylindre 107 disposé entre les extrémités 101, 104 de l'embase 99, muni de fentes traversantes 108, 109 pour le passage glissant des barres de transmission 105, 106. Les fentes sont dimensionnées en forme d'ouvertures arquées dans la direction circonférentielle du cylindre et d'angle A correspondant à l'angle de déplacement en pivot de la poignée selon l'axe J lors du mouvement d'ouverture/fermeture du mécanisme de l'ouvrant. Par exemple, l'angle peut être de 25 à 60 degrés. Le cylindre 107 comprend un alésage traversant central 110 dans l'axe J de section transversale non-circulaire, par exemple, carrée afin de recevoir un longeron 111 de section complémentaire à la section de l'alésage, qui est destiné à traverser l'ouvrant pour transmettre le couple de l'actionnement manuel de la poignée sur un mécanisme d'ouverture/fermeture de l'ouvrant. Le cylindre comprend des moyens de blocage en rotation pour bloquer le cylindre vis-à-vis du châssis. Ces moyens de blocage peuvent comprendre une clavette 112 configurée pour s'engager dans un logement 113 du châssis de la poignée afin de solidariser le cylindre avec le châssis lors du pivotement de ce dernier lors de l'actionnement de la poignée.

Le fonctionnement de l'organe de récupération d'énergie 96 est le suivant. Lorsqu'un utilisateur abaisse la poignée, le châssis tubulaire 97 solidaire de l'embase de préhension 4 pivote autour de l'axe J d'un angle A, par exemple 30 degrés. L'arbre du moteur solidaire de l'embase 99 elle-même solidaire de l'ouvrant reste stationnaire alors que le moteur lui-même solidaire du châssis tourne avec le châssis de 30 degrés autour de l'axe J. Ce mouvement induit un courant électrique dans le moteur qui est collecté par l'accumulateur électrique. La répétition des cycles d'ouverture et/ou fermeture permet de charger l'accumulateur et d'alimenter le moteur électrique des moyens d'actionnement. Le couple de l'action mécanique sur la poignée est transmis par le châssis au cylindre au travers de la clavette 112 et du cylindre au longeron 111 au mécanisme de l'ouvrant. Les fentes 108, 109 permettent le mouvement de rotation sans déformation du cylindre par rapport à l'embase fixe. Il va de soi que d'autres mode d'exécution sont possibles comme une inversion mécanique à l'exemple illustré comme par exemple un générateur mobile en rotation dans le châssis et un arbre solidaire du châssis.

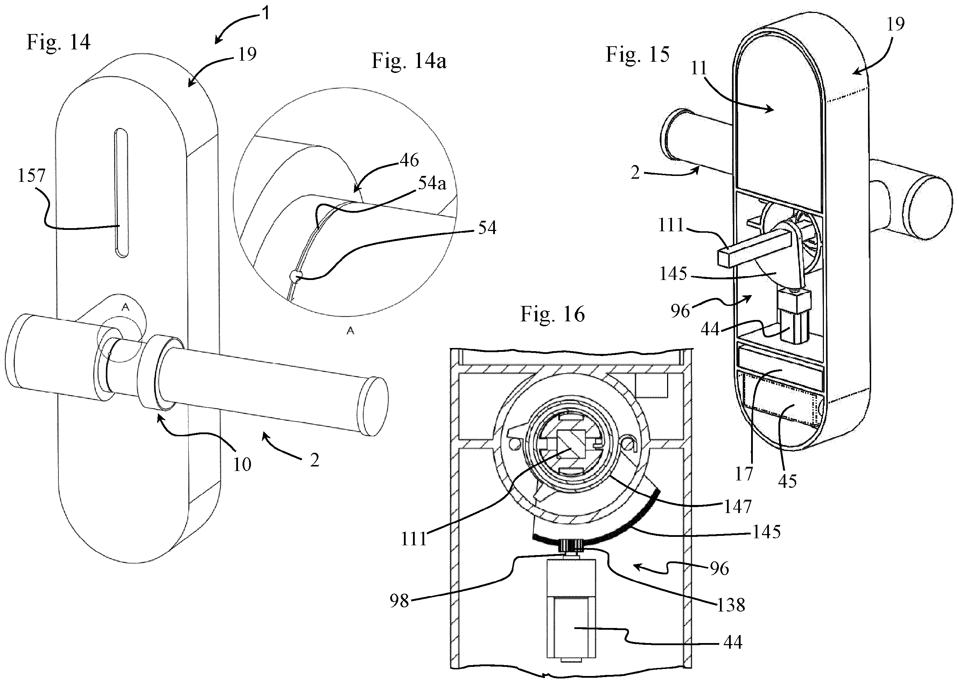

Les figures 15 et 16 illustrent un deuxième exemple d'organe de récupération d'énergie 96. L'organe comprend un générateur électrique 44, tel qu'une dynamo, connecté à un accumulateur d'énergie électrique 45, tel qu'une batterie rechargeable, logés à l'intérieur du boitier de poignée 19. L'arbre 98 du moteur comprend un pignon 138 engageant un engrenage 145, p.ex. sous forme d'une portion de disque dentée, couplée au longeron 111 pour transmettre le couple de l'actionnement manuel de la poignée sur le pignon 138. Cet engrenage transmet et amplifie le mouvement au pignon de petit diamètre. Le générateur électrique peut inclure un réducteur afin d'augmenter le rapport de transmission entre la rotation du longeron 111 et le rotor aimanté du générateur. Un ressort de rappel 147 retourne la poignée de sa position abaissée vers sa position de repos. Dans un mode de réalisation, le générateur électrique 44 est configuré pour charger l'accumulateur d'énergie électrique 45 dans les deux sens de rotation, toutefois il est également possible d'avoir le pignon 138 en roue libre dans le sens de retour de la poignée vers sa position de repos.

On peut ainsi rendre le système autonome en énergie en récoltant l'énergie mécanique de l'actionnement de la poignée de porte fournie par l'utilisateur en la convertissant en énergie électrique.

Un module électronique comprenant le contrôleur électronique 17 peut aussi être logé dans le boitier de poignée 19, servant au contrôle du moteur de l'organe d'application, et/ou à d'autres fonctions de contrôle du système, comprenant l'affichage de l'état du système ou l'émission d'alarmes. Dans le cas d'un système de désinfection mécanique, il est possible d'également envisager une petite récolte d'énergie pour alimenter le module électronique ou d'autres composants électroniques servant au contrôle du système ou d'affichage d'état.

Les moyens d'alimentation en liquide du dispositif de nettoyage de l'invention selon ce mode peuvent aussi comprendre une pompe 13 solidaire du châssis 97 et actionnée par l'effet du pivotement du châssis 97 autour de l'axe J lors de l'actionnement de la poignée. Pour cela, la pompe 13 peut comprendre un piston 114 logé dans une chambre 115 de la pompe actionnée en déplacement axial par un doigt 116 agencé au contact d'un plan incliné ou rampe solidaire d'une partie fixe de la poignée, par exemple du flasque 101. La pompe comprend de préférence un moyen de retour élastique pour le piston comme un ressort 120 dans la chambre. La pompe peut comprendre une valve d'admission antiretour 118 en communication avec le réservoir de liquide au moyen d'une portion de conduit et une valve de refoulement antiretour 119 reliée à une portion de conduite alimentant l'organe d'application. Les valves peuvent être chacune une valve à bille 121 ou pointeau comprenant un moyen de rappel élastique tel qu'un ressort hélicoïdal 122 ou tout autre type de valve unidirectionnelle. Le plan incliné 117 est configuré par rapport à la pompe de manière à agir sur la position axiale du doigt et donc du piston dans la chambre. En position d'appui du doigt, le piston comprime le liquide dans la chambre et le liquide est refoulé en direction de l'organe d'application. La valve de refoulement 119 est alors comprimée en ouverture pour laisser passer le liquide. La valve d'admission montée en sens à l'inverse à la valve de refoulement est alors fermée par l'action de rappel du ressort sur la bille. En position de retour du doigt, le piston est en position de détente dans la chambre. La valve d'admission est ouverte par la différence de pression crée entre la chambre et la conduite d'entrée ; ce qui provoque l'aspiration du liquide dans la chambre et son remplissage. La valve de refoulement reste fermée par le rappel élastique du ressort 122 sur la bille 121.

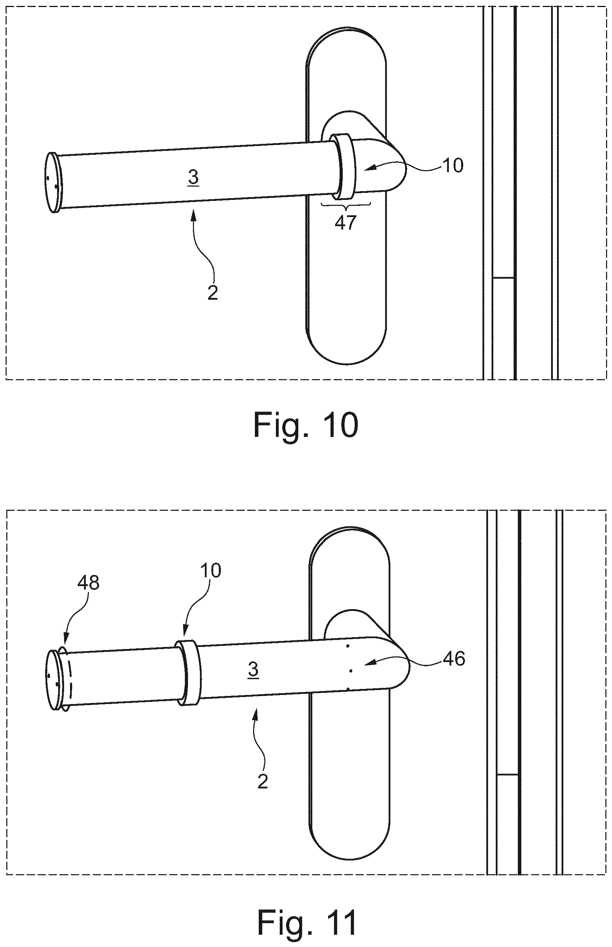

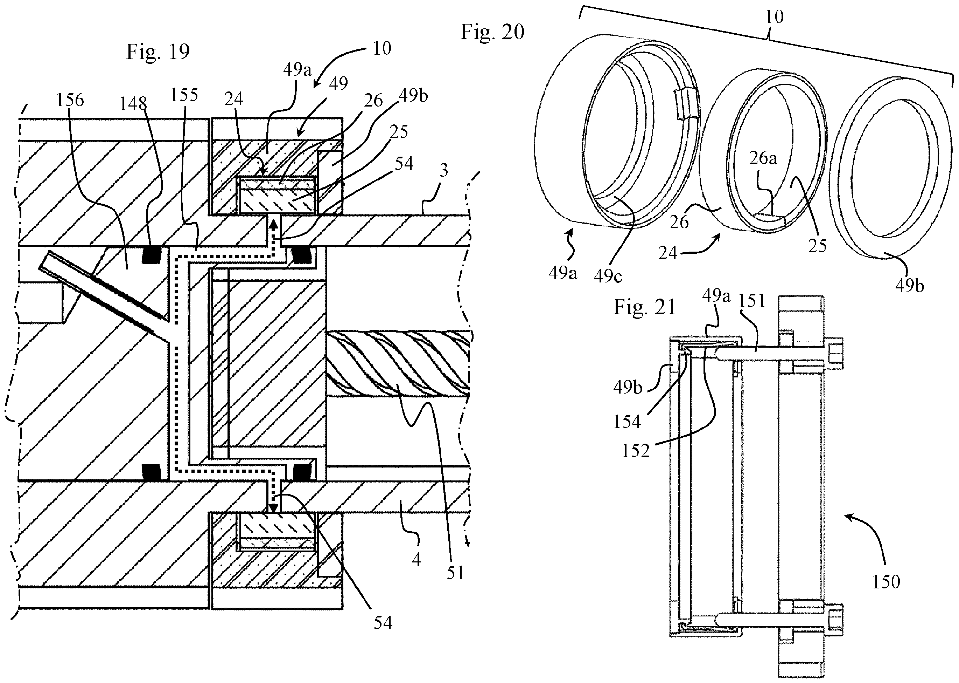

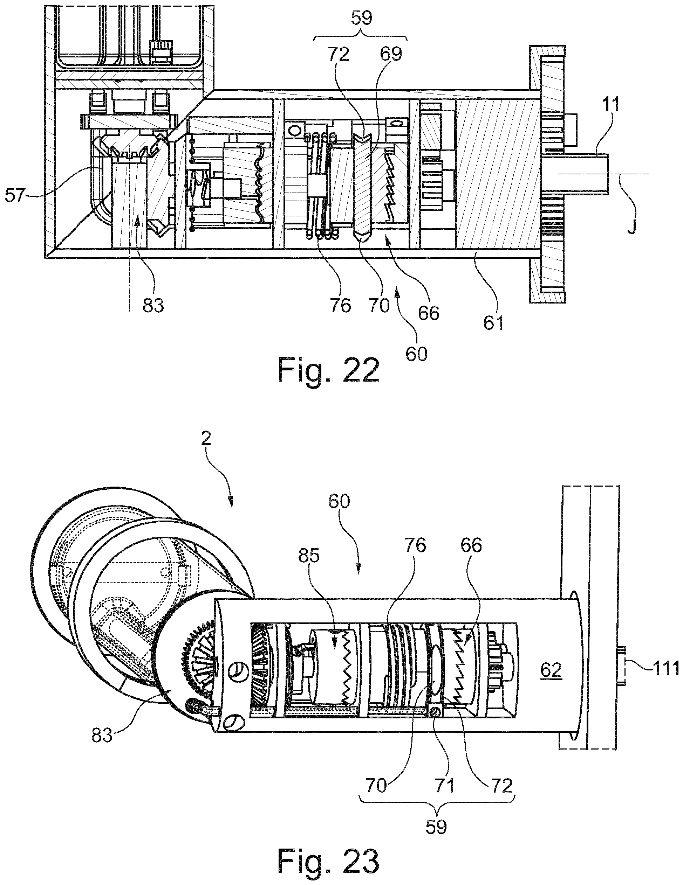

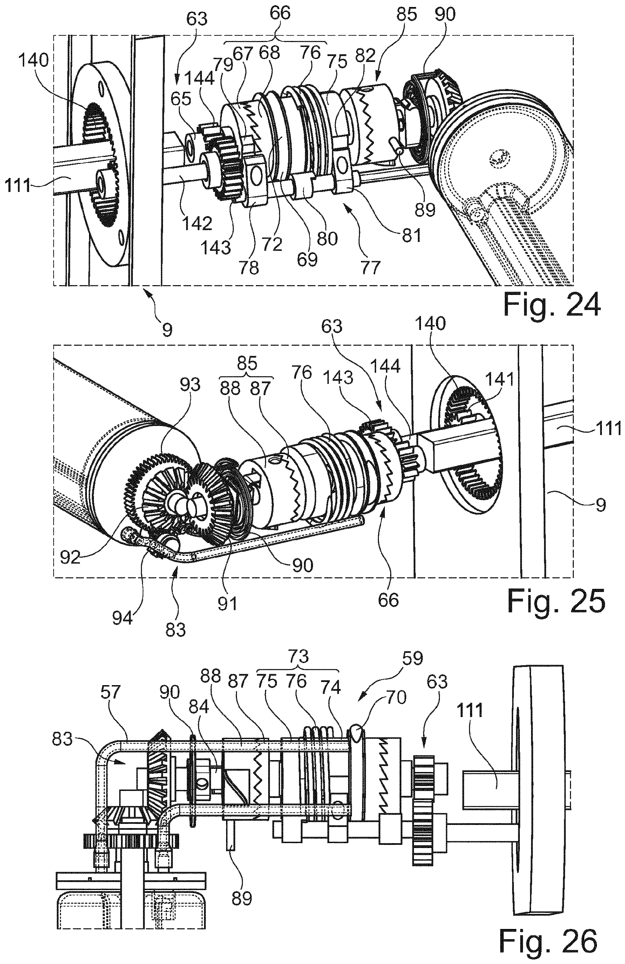

Un mode de réalisation préféré du dispositif selon l'invention est illustré en relation avec les figures 10 à 27. Dans ce mode, la poignée 2 comprend une surface de préhension 3 statique et un organe d'application 10 qui se déplace longitudinalement ou axialement (c.-à-d. dans la direction d'axe I) le long de la surface lors du nettoyage. Notamment, l'organe d'application 10 a la forme d'une bague montée autour de la surface 3 depuis une position d'alimentation dans laquelle la bague recouvre une zone d'alimentation 46 de la poignée pour l'alimentation du liquide dans l'organe comme le montre la figure 10. L'organe d'alimentation est déplacé par des moyens d'actionnement qui seront détaillés plus loin le long de la surface de préhension 3 comme le montre la figure 11. Le déplacement peut se faire entre la position d'alimentation 47 (figure 10) et une position de fin de course 48 correspondant à l'extrémité de la surface de préhension (figure 11 : position identifiée en ligne pointillée). Une fois cette position de fin de course atteinte par l'organe d'application, ce dernier est rappelé en position d'alimentation pour être rechargé en liquide nettoyant par des moyens d'actionnement qui vont être décrits ci-après.

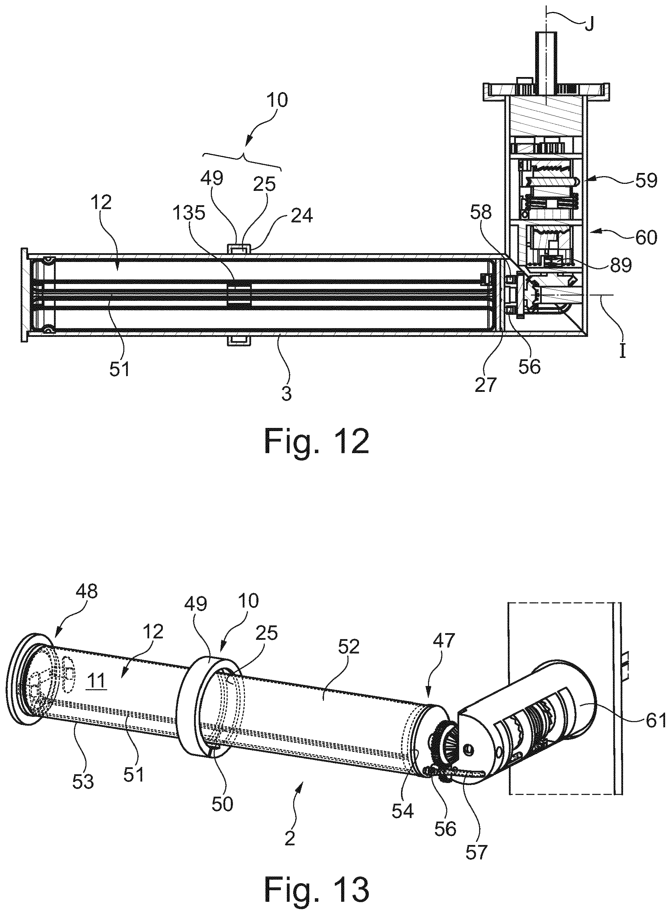

Comme le montrent les figures 12 et 13 et 18 à 21, l'organe d'application 10 comprend un tampon applicateur 25 disposé sur la surface interne d'un support annulaire rigide 49 de l'organe. Le tampon applicateur 25 est disposé de manière à rester en contact en permanence avec la surface de préhension 3 lors du déplacement axial de l'organe d'application.

Dans un mode de réalisation avantageux, le tampon applicateur 25 de l'organe d'application 10 est monté dans le support rigide 49 de manière démontable, afin de pouvoir remplacer ou nettoyer le tampon applicateur 25. De préférence, l'organe d'application 10 est agencé de manière à pouvoir enlever le tampon applicateur 25 de la poignée en laissant le support rigide 49 sur la poignée.

Au fil des utilisations, le tampon applicateur 25 se charge d'impuretés (poussière, film de gras laissé par la main de l'utilisateur...). Pour que la fonction de désinfection puisse continuer à s'effectuer efficacement, il est avantageux de remplacer régulièrement ce composant tampon par du personnel d'entretien. De ce fait, un système permettant un accès facile et rapide au composant tampon est nécessaire pour faciliter la tâche au personnel d'entretien. De plus, un système de verrouillage est avantageux à cet effet pour éviter que le composant ne se perde ou se fasse voler.

Le support rigide 49 peut être en deux parties 49a, 49b, par exemple comprenant une base 49a et un couvercle 49b monté de manière séparable de la base. Le tampon applicateur est verrouillé dans une rainure radiale du support rigide formée entre une flasque 49c de la base 49a et le couvercle 49b. Le couvercle peut être fixé par diverses manières à la base. Dans le mode de réalisation illustré, le couvercle est verrouillé à la base par un mécanisme de fixation 152, 154 élastique. Dans la variante illustrée, le mécanisme comprend un bras élastique 152 dans la base 49a engageant une épaule complémentaire 154 sur le couvercle. Il est également possible d'avoir un bras élastique sur le couvercle engageant une épaule sur la base. Un outil de déverrouillage 150 comprend une goupille 151 insérable dans le support annulaire rigide pour désengager le bras élastique de l'épaule complémentaire.

Dans une variante (non illustrée), les deux parties 49a, 49b de l'anneau 49 sont vissées ensemble.

Dans une variante, les deux parties 49a, 49b de l'anneau 49 sont assemblées avec un ou plusieurs aimants permanents 139b, par exemple situés dans l'épaulement de la base 49a, le couvercle 49b sous forme d'une rondelle venant se loger dans un recouvrement radial 49d. Le recouvrement 49d s'étend complètement autour du couvercle, ce qui rend la séparation des deux parties 49a, 49b à la main pratiquement impossible puisque l'utilisateur n'a aucune zone de préhension sur le couvercle. Un outil 150 avec une goupille 151 peut être utilisé pour venir pousser, à travers un ou plusieurs trous 149, le couvercle 49b, pendant que la base est maintenue, par exemple par la main du technicien d'entretien. Dans une variante, un outil avec un aimant permanent générant une force magnétique plus grande que la force magnétique de l'aimant 139b, peut être utilisé pour décoller le couvercle 49b de la base 49a.

Le tampon applicateur peut être sous forme d'un anneau fendu 26 flexible de sorte à pouvoir être ouvert en écartant les extrémités de la fente 26a et passer la poignée à travers la fente ouverte. Le tampon applicateur 25 peut être agencé pour exercer une petite compression élastique autour du tube de préhension 3 pour comprimer la matière tampon homogènement sur la surface de préhension et permettre un bon étalement du désinfectant.

Dans une variante, le tampon applicateur peut être en deux parties, par exemple deux moitiés.

Le tampon applicateur peut avoir diverses configurations, par exemple formé d'une pièce unique, ou formé d'un support annulaire 25a rigide mais flexible supportant un feutre, mousse ou autre matériel d'application 25b disposé sur la surface radialement intérieure de l'anneau flexible. Une bandelette constituée d'un matériau tampon 25b (de type feutre, éponge etc.) peut être collée sur la périphérie intérieure d'un anneau de matériau de support élastique, par exemple en plastique, pour être en contact avec le tube de préhension. Le rôle de cette bandelette, dans un premier temps, est d'être imbibée de liquide désinfectant pendant le processus d'irrigation. Dans un deuxième temps, le liquide imbibé est distribué le long du tube de préhension pendant la translation de l'anneau.

Optionnellement, une deuxième bandelette (non illustrée) peut être collée à l'intérieur de l'anneau de support, axialement à la bandelette tampon avec un petit espacement. Cette deuxième bandelette joue le rôle de racleur permettant de récupérer le surplus de désinfectant déposé sur le tube de préhension. Ceci permet d'éviter qu'un utilisateur ne touche une poignée mouillée, ce qui peut être perçu comme désagréable et par la même occasion, éviter que des personnes à la peau particulièrement sensible ne rentrent en contact avec le liquide désinfectant. Cette bandelette peut être d'un matériau de type éponge, feutre, polymère ou caoutchouc.

Dans un mode de réalisation, le support annulaire 49 est relié à la poignée par une base 50 comprenant un écrou 135 lequel est monté et déplacé sur une vis sans fin 51 logée à l'intérieur de la paroi partiellement tubulaire 52 de la poignée et s'étendant au moins sur la largeur de la surface de préhension 3. La paroi 52 comprend un passage longitudinal traversant 53 s'étendant axialement pour permettre à la base du support annulaire de l'organe de se déplacer longitudinalement dans le passage entre la position d'alimentation 47 et la position de fin de course 48.

Dans la position d'alimentation 47 est disposé un collecteur de distribution en liquide 27 configuré avec plusieurs sorties de liquide 54 distribuées radialement et préférablement sensiblement alignées dans un plan circonférentiel de la surface de préhension de manière à être recouvertes par le tampon applicateur 25 lorsque l'organe d'application est en position d'alimentation. Le collecteur de distribution 27 est de préférence alimenté par un connecteur 56 reliée à un conduit 57 lequel est relié à un réservoir de liquide 11.

Dans un mode de réalisation, des sorties de liquide 155 de la structure interne 156 sont alignées avec des trous radiaux 54 du tube de préhension, des joints 148 dans un matériau élastomère étant logés autour des sorties 155 pour garantir une bonne étanchéité entre le tube de préhension et la structure interne 156.

Une fine rainure 54a circulaire intersectant les trous de distribution 54 peut être présente pour rendre l'irrigation de la zone tampon plus homogène pour une désinfection efficace sur l'intégralité de la surface de préhension 3.

Dans un mode de réalisation, le réservoir est logé à l'intérieur de la poignée comme dans la paroi partiellement tubulaire 52 comprenant un connecteur de sortie 58 reliée au conduit 57.

Dans un autre mode de réalisation, le réservoir 11 est logé dans un boitier de poignée 19 fixé à l'ouvrant, comme illustré dans les figures 14 et 15. Le boitier peut comprendre une fenêtre 157 dans sa face avant, agencée pour permettre de voir le niveau de remplissage du réservoir 11. Une jauge avec éventuellement la présence d'un élément flottant coloré peut être utilisée pour faciliter la visualisation du niveau du liquide.

Dans d'autres modes de réalisation, le réservoir peut aussi être sous forme d'un réservoir indépendant de la poignée ou du boitier de poignée 19, et connecté à la poignée 2 par une conduite. Le réservoir 11 peut être une cartouche amovible échangeable ou un réservoir fixe rechargeable, par exemple logée dans la poignée 2 ou le boitier de poignée 19. Une pompe 59 est disposée entre le réservoir et le collecteur pour alimenter l'organe d'application en liquide avant chaque cycle de nettoyage. La pompe peut être une pompe de type péristaltique qui comprime le conduit 57 souple et élastique. Toutefois, d'autres types de pompe peuvent être prévues comme une pompe à piston, à diaphragme ou autre.

Dans le mode de réalisation préféré, le dispositif est autonome en énergie pour l'alimentation du liquide par la pompe 59 et pour le déplacement de l'organe d'application lors du cycle de nettoyage. De plus, le dispositif est essentiellement mécanique de sorte qu'il peut être disposé contre l'ouvrant sans recours nécessaire à une alimentation électrique.

Pour cela, le dispositif comprend un organe d'accumulation d'énergie mécanique et de restitution et de transmission d'énergie mécanique 60 (appelé « organe d'énergie et d'entraînement » par la suite) à l'organe d'application dont le principe donné à titre d'exemple préférentiel est le suivant. L'organe d'énergie et d'entraînement 60 est ainsi configuré pour emmagasiner l'énergie mécanique d'actionnement de la poignée lors de son pivotement autour de l'axe J lors de l'abaissement de celle-ci pour l'ouverture de l'ouvrant. Ainsi, la poignée comprend un châssis tubulaire 61 de réception de l'organe d'énergie et d'entraînement 60 et de liaison cinématique entre le mécanisme de fermeture de la porte et l'organe d'application. Le châssis 61 de l'organe est sensiblement perpendiculaire à la paroi tubulaire 52 et à la paroi de la surface de préhension et comprend un bâti 62 logé à l'intérieur du châssis tubulaire sur lequel est monté en amont un mécanisme de multiplication de vitesse 63 relié au longeron 111 du mécanisme de fermeture de la porte. Le mécanisme de multiplication de vitesse 63 est configuré pour entraîner un arbre 65 de sortie du mécanisme sur un peu plus de 360 degrés lorsque le longeron 111 du mécanisme de fermeture de la porte est sollicité en pivotement selon un angle inférieur à 180 degrés (par exemple environ de 20° à 40°) correspondant à la course de l'abaissement de la poignée lors de l'ouverture de la porte. Le mécanisme de multiplication amont 63 est constitué d'un engrenage de roues et pignons dont la conception est à la portée de l'homme du métier. En particulier, le mécanisme 63 comprend une couronne dentée 140 formée dans la paroi de l'ouvrant 9 dans laquelle s'engrène un pignon satellite 141. Le pignon 141 est monté sur un arbre 142 qui porte un engrenage formé d'un premier pignon 143 qui entraîne un second pignon 144 monté sur l'arbre de sortie 65 du mécanisme. Le mécanisme 63 transmet ainsi un couple à un premier embrayage unidirectionnel ou antiretour 66 formé d'une partie amont crantée 67 et une partie aval crantée 68 capables de transmettre un couple dans la direction aval seulement. Les deux parties crantées 67, 68 coopèrent entre une position accouplée dans laquelle le couple est transmis de la partie crantée amont 67 à la partie crantée 68 et une position désaccouplée où aucun couple n'est transmissible en retour de la partie crantée aval 68 à la partie crantée amont 67. L'effet antiretour de l'embrayage est obtenu par un engagement asymétrique des dents des parties crantées 67, 68, configuré de telle manière à créer un accouplement mécanique que dans une seule direction en rotation et un désaccouplement dans la direction opposée. La partie aval 68 de l'embrayage est solidaire de la pompe péristaltique 59 qui comprend une partie de disque rotatif 69 entraînée par le couple transmis par l'embrayage avec une rainure annulaire 72 dans la partie de disque ainsi qu'un passage tubulaire rigide fixe 71 dans le bâti pour le passage du conduit flexible. La zone de passage comprend une came 70 qui comprime périodiquement le tube lors de chaque rotation de la partie rotative 69. La partie rotative 69 est elle-même solidaire d'un ensemble 73 de loquets cycliques comprenant un loquet amont 74 en forme de roue et un loquet aval 75 en forme de roue reliés par un premier ressort principal de torsion 76. Il faut toutefois noter que la rainure annulaire 72 est préférablement omise de façon à faciliter le couplage et découplage de l'embrayage à effet antiretour. L'activation des loquets est réglée par un arbre à came 77 disposé le long de l'organe. L'arbre à came 77 comprend une première dent 78 disposée sur l'arbre et à proximité de la partie amont 67 de l'embrayage pour engager un doigt 79 disposé sur la surface de de cette partie.

L'arbre à came 77 comprend aussi une deuxième dent 80 sur lequel peut prendre appui un ressort de torsion 76 pour éviter à celui-ci de se détendre lorsque la poignée revient en position de repos (de préférence horizontalement). Enfin l'arbre 77 comprend une troisième dent 81 qui s'engage cycliquement sur un doigt 82 du loquet aval 75 de manière à libérer le loquet lorsque la première dent 78 est en prise avec le doigt 79 et donc restituer l'énergie du ressort en aval sur une transmission 83 reliée à la vis sans fin qui déplace l'organe d'application.

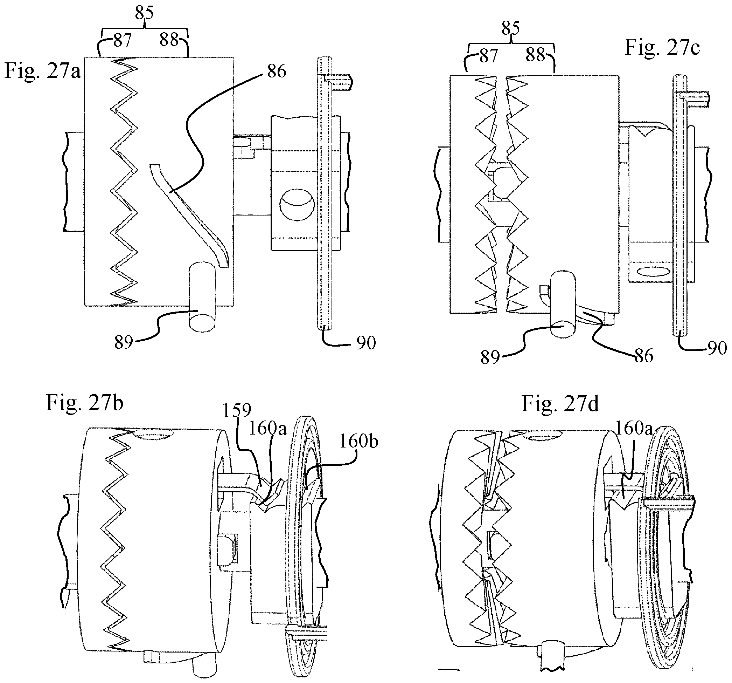

La transmission 83 est entraînée par un arbre 84 relié à l'ensemble 73 de loquets par l'intermédiaire d'un second embrayage cyclique bidirectionnel 85 constitué de deux parties crantées 87, 88. Ce second embrayage est configuré pour transmettre un couple à la transmission 83 en position accouplée pour le déplacement de l'organe d'application sur la surface de préhension, en aller dans la position de fin de course et en position désaccouplée lors du retour dans la position d'alimentation lors du nettoyage. Le passage à la position désaccouplée du second embrayage 85 est effectué au moyen d'un chemin de came 86 porté par la surface de la partie aval 88 et d'un doigt 89 supporté par le bâti de la poignée. Les positions couplée (figures 27a, 27b) et désaccouplée (figures 27c, 27d) peuvent avoir des positions stables créées par une élément élastique sur une partie, tel qu'une languette élastique 159, engageant une protubérance ou creux 160a, 160b sur l'autre partie, dans les deux positions stables.

L'organe d'énergie et d'entraînement comprend un second ressort de torsion 90 relié à l'arbre de transmission 84 et au bâti afin de procurer l'énergie nécessaire au retour de l'organe d'application de sa position de fin de course à sa portion d'alimentation. L'énergie de ce second ressort est emmagasinée lors de la phase aller de l'organe d'application et de restitution de l'énergie du premier ressort 76. Ce second ressort 90 se tend à mesure que le premier ressort 76 se détend. Le second ressort 90 est choisi avec une constante de raideur plus faible que le premier ressort 76 de façon à permettre l'emmagasinement d'une partie de l'énergie par le second ressort. Le second ressort pourrait être remplacé par tout autre type de ressort comme un ressort linéaire ayant une force de rappel constante ou proportionnelle fixé, par exemple directement à la bague de support 49. Les deux ressorts 76 et 90 pourraient eux-mêmes être des ressorts à couple constant ou quasi-constant.

La transmission ou multiplicateur 83 en sortie de l'organe d'énergie et d'entraînement peut être réalisée par un engrenage d'au moins deux pignons 91, 92 arrangés à 90 degrés ou tout angle approprié et éventuellement un engrenage secondaire avec une roue 93 coaxiale au pignon de sortie 92 et engrainant un pignon 94 couplé à la vis sans fin. Toute autre transmission appropriée permettant l'entraînement et le réglage précis de la course de la bague entre les deux positions de course 46, 48 de la bague est possible.

Selon un aspect avantageux de l'invention, la transmission est une transmission par un couplage magnétique. L'écrou d'entrainement 135 entraine un aimant 139 qui lui entraine la bague de l'organe de nettoyage 10, celle-ci comprenant un corps aimanté ou ferromagnétique par exemple logé à l'intérieur de la bague. Un rail 158 disposé dans la structure interne 156 engage un guidage complémentaire dans l'écrou 135 afin de bloquer la rotation de l'écrou et assurer son déplacement en translation uniquement.

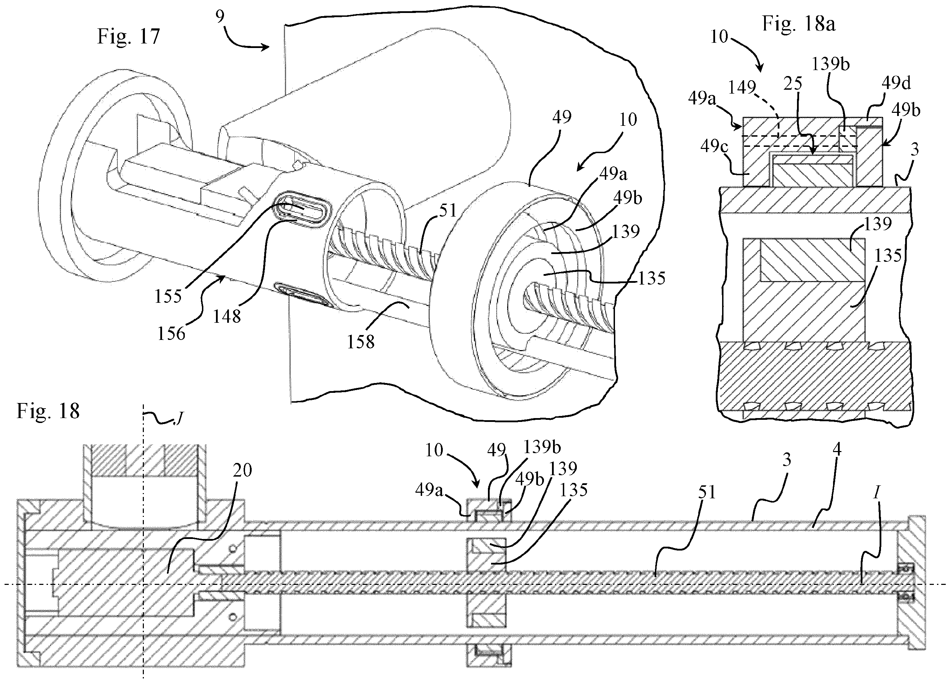

Le support annulaire rigide (bague) 49 peut par exemple comprendre ou être constitué d'un matériau ferromagnétique, ou d'un aimant ayant une polarisation complémentaire à la polarisation magnétique de l'aimant permanent 139. Dans l'exemple illustré dans la figure 18, le support annulaire rigide 49 comprend un aimant permanent 139b logé entre deux parties 49a, 49b ferromagnétiques du support annulaire 49. La polarisation de l'aimant 139b sur la bague 49 est opposée à la polarisation de l'aimant 139 sur l'écrou dans la direction de translation (direction de l'axe *I* de l'écrou).

L'embase tubulaire 4 de la poignée est en un matériau non-magnétique.

Dans un mode de réalisation comprenant un aimant permanent sur l'organe d'application 10, il est aussi envisageable d'avoir un écrou ferromagnétique ou ayant une partie ferromagnétique au lieu d'un aimant.

Afin d'éviter les phénomènes d'arcboutement, la bague peut être couplée à une multitude d'aimants répartis de manière homogène dans la bague ainsi que dans le périmètre interne de la surface de préhension translaté par un ou plusieurs actuateurs linéaires (vis - écrou ou courroie).

Dans un mode de réalisation tel qu'illustré, le support annulaire rigide 49 comprend des flasques 49a, 49b aux extrémités axiales du support, le couplage magnétique des flasques à l'écrou permettant aussi d'éviter l'arcboutement de la bague.

Ce couplage magnétique offre une sécurité pour le mécanisme puisqu'une translation de l'anneau par l'utilisateur n'a aucun impact sur le mécanisme situé dans la poignée de porte. De plus, si dans un cas particulier l'utilisateur garde sa main sur la poignée durant la phase de désinfection, l'anneau bloqué par la main de l'utilisateur se découple de l'écrou et ne gêne pas l'utilisateur : l'anneau n'exerce pas de force sur la main qui est restée sur la poignée.

Le fonctionnement général de l'organe d'énergie et d'entraînement est le suivant. Lors de l'abaissement de la poignée 2, le premier embrayage unidirectionnel 66 est en position d'accouplement. Le loquet amont 74 est en position libre alors que le loquet aval 75 est en position bloquée. L'ensemble de loquets cyclique 73 isole ainsi le ressort de torsion principal 76 qui se tend et emmagasine l'énergie mécanique du mouvement d'abaissement de la poignée. Le second embrayage bidirectionnel 85 est de préférence en position accouplée. Lors de cette phase, l'organe d'application 10 est en position d'alimentation en vis-à-vis du collecteur de distribution de liquide. Lors de cette phase de rotation sur un tour environ, la pompe péristaltique 59 est aussi actionnée en rotation ce qui engage la came 70 sur le conduit de sorte que le liquide est pompé du réservoir vers le tampon applicateur remplissant ainsi celui-ci en liquide de nettoyage.

Lorsque la poignée est désengagée et remonte de sa position abaissée, le premier embrayage antiretour 66 se désaccouple et l'ensemble de loquets est bloqué par le mécanisme 81, 82 de l'arbre à came. Arrivé à la position de déplacement finale de la poignée, le doigt 79 vient pousser les dents 78 et 81, libérant ainsi le doigt 82 et le premier ressort de torsion 76. Le premier ressort de torsion 76 est alors libéré et le doigt 82 de la roue aval 75 du loquet est libérée ce qui permet la transmission du couple emmagasiné par la détente du ressort à la transmission ou multiplicateur aval 83. L'organe d'application est par conséquent déplacé de la position d'alimentation à la position de fin de course permettant ainsi le nettoyage de la poignée. Lors de cette phase, l'embrayage est en position accouplé et le premier ressort 76 tend le second ressort 90 qui emmagasine donc de l'énergie dans cette phase aller de l'organe 10.

Un fois l'organe d'application 10 arrivé en position de fin de course, le second ressort est tendu à sa tension maximale. La roue aval 75 du loquet cyclique 73 n'est alors pas encore en position bloquée. Le second embrayage cyclique 66 se désaccouple à la fin de cette première rotation, la roue avale 75 n'est pas encore bloquée. Le second embrayage cyclique 85 se désaccouple alors (le doigt 89 engagé dans le chemin de came 86 sépare éloigne les parties crantées 87, 88 de l'embrayage l'une par rapport à l'autre). Le second ressort 90 se détend ce qui entraîne la transmission en sens opposé et entraine l'organe d'application 10 qui retourne en position d'alimentation.

Une fois l'organe en position d'alimentation, la poignée est au repos et un nouveau cycle d'alimentation et de nettoyage peut recommencer lorsque la poignée est à nouveau abaissée par un utilisateur.

Il faut noter que le dispositif peut fonctionner avec deux poignées opposées et disposées de part et d'autre de l'ouvrant. Le dispositif est parfaitement autonome en énergie et cette énergie est renouvelable puisqu'elle n'a pour origine que la force musculaire.

Il faut noter que l'organe d'énergie et d'actionnement du mode des figures 10 à 27 pourrait être toutefois remplacé une unité d'actionnement comprenant un contrôleur électronique, un moteur électrique d'actionnement ou en utilisant un moteur linéaire, optionnellement un ou plusieurs capteurs de position de la bague annulaire, et optionnellement aussi un organe de récupération d'énergie de la poignée, de transformation et de stockage en énergie électrique pour l'alimentation des éléments électriques et électroniques du dispositif.

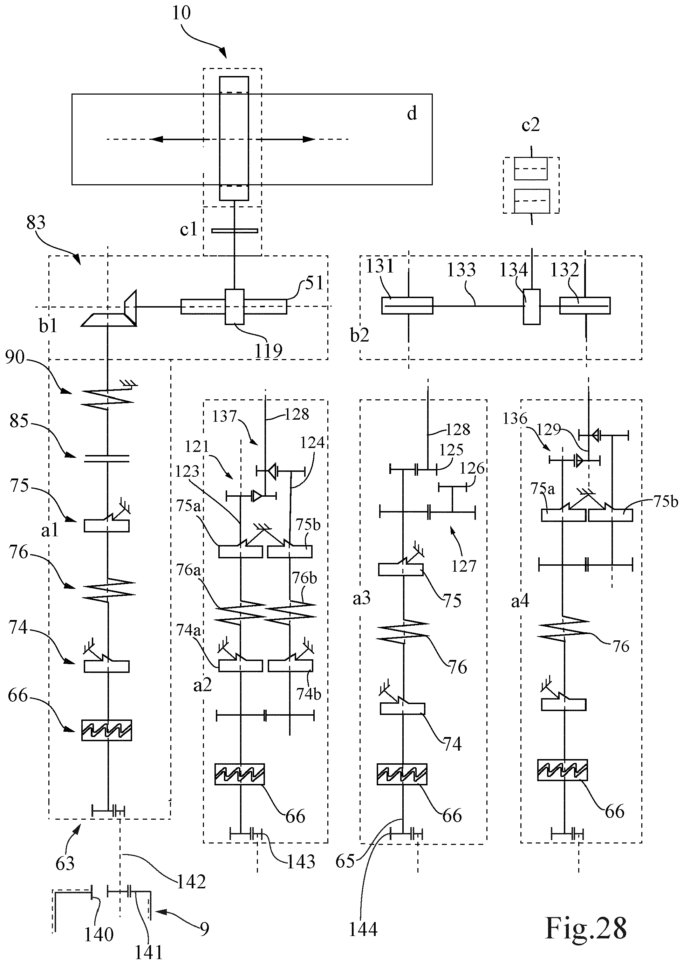

La figure 28 représente plusieurs combinaisons possibles de modules du dispositif avec stockage et restitution d'énergie mécanique.

D'une manière générale, le dispositif comprend un organe d'accumulation et de restitution d'énergie mécanique sous forme d'un bloc de stockage et d'inversion a1, a2, a3 ou a4. Le bloc de stockage et d'inversion est relié à un bloc de translation b1 ou b2 et à un bloc de couplage c1 ou c2 pouvant être relié à l'organe d'application 10 ou la surface de préhension 3. Les blocs de stockage et d'inversion a1-a4, de translation b1-b2 et de couplage c1-c2 peuvent être sélectionnés selon différentes combinaisons possibles.

Selon une première configuration du dispositif, le bloc de stockage et d'inversion a1, le bloc de translation b1 et le bloc de couplage c1 font partie du dispositif décrit dans le mode des figures 18 à 27. Les mêmes références sont utilisées pour désigner les mêmes éléments.

D'autres modes de réalisation sont envisageables, les blocs de stockage et d'inversion a1, a2, a3 et a4 étant interchangeables de même que les blocs de translation b1 et b2 ainsi que les blocs de couplage c1 et c2.

A titre d'exemple une combinaison a2 - b1 - c2 est une solution possible. Les réducteurs et multiplicateurs de vitesse ainsi que les mécanismes d'ouverture des dents des loquets ne sont pas représentés dans cette figure. Chaque élément est représenté de manière schématique, sans ancrage à une référence et sans souci d'échelle.

Le bloc de stockage et d'inversion a2 est constitué (par ordre d'actionnement) d'un embrayage anti retour couplé en sortie au premier ressort 76a qui est lui-même bloqué par un système de loquets/dents 75a en entrée et en sortie de celui-ci, tout comme pour le bloc a1. Ce système permet au ressort d'être armé lors de la descente de la poignée sans être désarmé lors de la remontée de celle-ci. Parallèlement, un second système de ressort bloqué 76b, entre deux loquets/dents 74b, 75b est lui aussi armé lorsque l'utilisateur actionne la poignée en pivotement (par exemple pour la descendre lors de l'ouverture/fermeture de l'ouvrant). Deux engrenages unidirectionnels 136, 137 (couplés dans un sens de rotation, en roue libre dans l'autre sens de rotation) sont disposés en sortie d'arbres de manière à ce que le premier arbre 123 entraine l'anneau dans sa phase allé tandis que le second arbre 124 entraine l'anneau dans sa phase de retour, sans couplage de l'arbre 124 lors de la phase allée et sans couplage de l'arbre 123 dans la phase retour.

Lorsque la dent du loquet 75a de sortie du premier ressort 76a est ouverte, ce dernier se décharge entrainant l'anneau de l'organe d'application 10 sur sa phase aller. Une fois arrivé en bout de course, la dent du loquet de sortie 75b du second ressort 76b est à son tour ouverte, entrainant l'anneau dans sa phase de retour. Les dents des loquets sont cycliquement ouvertes par un système d'arbre à cames mû par l'arbre central (non illustré). Ils sont généralement passivement fermés par des ressorts de rappels immédiatement après avoir été ouverts.

Le bloc de stockage et d'inversion a3 est très similaire au bloc a1, à l'exception près que l'inversion du sens de rotation se fait avec un moyen d'inversion similaire à un système de boite à vitesse. L'anneau de l'organe d'application passe de la phase aller à la phase retour lorsque le pignon 125 lié à l'arbre de sortie 128 est déplacé relativement en translation pour venir se coupler au pignon 126 du deuxième arbre 127, qui lui tourne dans la direction opposée. Dans ce cas le ressort doit assurer l'aller et le retour de l'anneau lors de sa détente.

Le bloc de stockage et d'inversion a4 est une combinaison des blocs a2 et a3. L'unique ressort 76 sert ici à mouvoir l'anneau de l'organe d'application dans sa phase aller et retour tout comme pour le bloc a3. Toutefois, le moyen d'inversion du sens de rotation fait par translation du pignon de sortie 129 pouvant être plus complexe qu'un double système d'anti retour, cette dernière solution est appliquée ici tout comme dans le bloc a2.

Le bloc de translation b2 représente une translation de l'anneau nettoyant faite par un système poulies 131, 132 et courroie 133.

Le bloc de couplage c2 représente un couplage magnétique. L'écrou 135 de la vis sans fin ou de l'élément de connexion 134 est solidaire d'un aimant ; tous deux se translatant dans un système clos. L'anneau dans lequel est inséré un système d'aimants ou de corps ferreux, est alors couplé magnétiquement à l'aimant de l'écrou 135 ou de l'élément 134. Plusieurs blocs d'aimants c2 et de translation (b1 ou b2) peuvent être nécessaires pour que l'anneau soit correctement guidé.

De nombreuses autres versions peuvent être imaginées stockant par exemple l'énergie lors de la descente et de la remontée de la poignée, composés aussi de systèmes d'anti retour, d'inverseurs, et de loquets à dents similaires à ceux présentés dans les blocs a1, a2, a3 et a4.

### Liste des références dans les figures

ouvrant 9
   mécanisme d'ouverture (non visible)
   paroi de l'ouvrant (non représenté)
dispositif de nettoyage 1
   boitier de poignée 19
      fenêtre 157
   ressort de rappel 147
   **poignée 2**
      structure interne 156
         sorties de liquide 155
         rail 158
      tube de préhension / surface de préhension 3 embase 4
         portions d'extrémités 5, 6
         noyau central 7
      axe de pivot 8
      plan incliné 117
      zone d'alimentation 46
         position d'alimentation 47
         collecteur de distribution en liquide 27
            sorties de liquide 54
               orifices 54
               rainure 54a
            joint 148
      position de fin de course 48
      paroi partiellement tubulaire 52
         passage longitudinal traversant 53
      châssis tubulaire 61
         bâti 62
   **organe d'application / de nettoyage 10**
      **(sous forme de bague)**
      réserve tampon 24
         tampon applicateur 25
         anneau fendu 26
            fente 26a anneau support matériau / bandelette tampon
      support annulaire rigide 49
         base 49a
            flasque 49c
            recouvrement radial 49d
            trou 149
         couvercle 49b
            mécanisme de fixation 152, 154 déverrouillable
            base 50
               écrou 135
   **réservoir 11 de liquide**
      solution de nettoyage
   **moyens d'alimentation 12 (du liquide)**
      pompe à liquide 13
         piston 114
         chambre 115
         ressort 120
         doigt 116
         valve d'admission antiretour 118
         valve de refoulement antiretour 119
            bille 121
            ressort hélicoïdal 122
      conduite 14
      connecteur 56
      conduit 57
      connecteur de sortie 58
      pompe 59 (pompe péristaltique)
         partie de disque rotatif 69
            rainure annulaire 72
            came 70
         passage tubulaire rigide fixe 71
   **capteur 15 (de présence)**
   **unité d'actionnement 16**
      contrôleur électronique 17
      moyens d'entraînement 18
         moteur électrique 20
         vis sans fin 51
         écrou 135
            aimant permanent 139
   **organe de récupération d'énergie 96**
      générateur de conversion d'énergie mécanique en énergie électrique 44
         arbre de transmission 98
            pignon 138
            cylindre 107
               fentes traversantes 108, 109
               alésage traversant central 110
         engrenage 145 (p.ex. portion de disque denté)
         embase 99
            flasque 101
               trous 102
            pièce de raccordement 103
            extrémité proximale 104
            barres de transmission, par exemple deux barres parallèles 105, 106
      mécanisme de transmission de couple 64
      accumulateur d'énergie électrique 45
      châssis tubulaire 97
         logement 113
      clavette 112
      longeron 111
   **organe d'énergie et d'entraînement 60**
      mécanisme de multiplication de vitesse 63
         arbre 65 de sortie
         couronne dentée 140
         pignon satellite141
         arbre 142
         premier pignon 143
         second pignon 144
      **premier embrayage unidirectionnel ou antiretour 66**
         partie amont crantée 67
         partie aval crantée 68
      ensemble 73 de loquets cycliques
         loquet amont 74
         loquet aval 75
            doigt 82
         premier ressort principal de torsion 76
         arbre à came 77
            première dent 78
            deuxième dent 80
            troisième dent 81
               doigt 79
      transmission 83
      arbre 84
      **second embrayage cyclique bidirectionnel 85**
         partie aval 88
         doigt 89 de came
         chemin de came 86
         languette élastique 159
         second ressort de torsion 90
      pignons 91, 92 arrangés à 90 degrés
      roue 93
      pignon 94
**organe d'accumulation et de restitution d'énergie mécanique**
   bloc de stockage et d'inversion a1, a2, a3 ou a4
   bloc de translation b1 ou b2
   bloc de couplage c1 ou c2
   engrenages unidirectionnels 136, 137
   premier arbre 123
   second arbre 124
   pignon 125
   arbre de sortie 128
   pignon 126
   deuxième arbre 127
   pignon de sortie 129
   système poulies 131, 132 et courroie 133
   élément de connexion 134
**outil de déverrouillage 150**
   goupille 151
première étape 100
seconde étape de détection de l'arrêt de l'action sur la poignée 200
l'étape suivante 300
l'étape suivante 400
*axe longitudinal J du châssis (axe de pivotement de la poignée)*
*axe I*
*angle A*

## Revendications

1. Dispositif de nettoyage d'une poignée (1) comprenant au moins une poignée (2) comprenant une surface de préhension (3) à nettoyer faisant partie d'une paroi de forme tubulaire ou partiellement tubulaire, un organe d'application (10) pour appliquer un liquide nettoyant sur la surface de préhension (3), et des moyens d'alimentation (12) en liquide nettoyant comprenant au moins un réservoir (11) pour recevoir et distribuer du liquide nettoyant à l'organe d'application, l'organe d'application (10) formé en partie au moins d'une bague annulaire montée autour de la surface de préhension (3) et comprenant au moins une réserve tampon (24) agencée au contact de la surface de préhension (3), le dispositif comprenant en outre une unité d'actionnement (16) de l'organe d'application comprenant des moyens d'entraînement configurés pour déplacer l'organe d'application (10) axialement le long de ladite surface de préhension (3) entre une position de distribution, dans laquelle l'organe d'application est en vis-à-vis du collecteur de distribution, ou communication fluidique avec celui-ci, pour le transfert de liquide nettoyant dans la réserve tampon (24) et une configuration d'application pour l'application de liquide nettoyant sur la surface de préhension (3) de façon à appliquer du liquide nettoyant depuis la réserve tampon (24) sur la surface de préhension lors de ce déplacement, la réserve tampon (24) comprenant un tampon applicateur (25) en contact avec la surface de préhension (3) ayant une structure permettant de stocker le liquide dans une structure poreuse ou d'interstices de rétention du liquide, tel **que** le dispositif de nettoyage comprend un collecteur de distribution (27) de liquide nettoyant comprenant au moins une entrée de liquide en communication avec le réservoir de liquide et une pluralité de sorties (54) de liquide distribuées en regard du tampon applicateur (24) .

2. Dispositif selon la revendication 1, **caractérisé en ce que** les moyens d'entraînement sont configurés pour déplacer l'organe d'application (10) par un couplage magnétique.

3. Dispositif selon la revendication 2, **caractérisé en ce que** les moyens d'entraînement comprennent un système poulies (131, 132) et courroie (133) dans la poignée comprenant un élément de connexion (134) solidaire d'un aimant.

4. Dispositif selon la revendication 2, **caractérisé en ce que** les moyens d'entraînement comprennent une vis sans fin s'étendant longitudinalement à l'intérieur de paroi tubulaire de la surface de préhension de la poignée et un écrou déplaçable le long de la vis sans fin, l'écrou entrainant un aimant (139) qui entraine la bague de l'organe de nettoyage, celle-ci comprenant un corps aimanté ou ferromagnétique (49a, 49b, 139b).

5. Dispositif selon l'une quelconque des revendications précédentes, **caractérisé en ce que** l'unité d'actionnement (16) comprend un contrôleur électronique (17) configuré pour commander automatiquement l'action des moyens d'entraînement (18, 32) pour le déplacement relatif de la réserve tampon (24) avec la surface de préhension (3) et/ou commander la distribution en liquide par les moyens d'alimentation (12) pour remplir en liquide la réserve tampon de l'organe d'application à partir du réservoir de liquide (12).

6. Dispositif selon la revendication précédente, **caractérisé en ce qu'**il comprend au moins un capteur (15) agencé pour détecter la présence du touché de la poignée par un utilisateur, ledit capteur étant associé au contrôleur électronique (17) lequel est configuré pour actionner automatiquement les moyens d'alimentation (12) du liquide et/ou d'entraînement (18, 32), en réponse à une information de détection.

7. Dispositif selon l'une quelconque des revendications précédentes, **caractérisé en ce qu'**il comprend au moins un bloc de stockage et d'inversion (a1-a4) ou organe de récupération d'énergie mécanique (96, 60) par des moyens de transmission de couple (63, 104, 140-144) du fait de la rotation de la poignée sur un mécanisme d'ouverture et/ou fermeture d'un ouvrant (9), pour le stockage de cette énergie mécanique et/ou de transformation et stockage en énergie électrique (45) et enfin de redistribution de cette énergie mécanique et/ou électrique stockée à l'unité d'actionnement (16) ou aux moyens d'entraînement (18, 32 ; 51, 60) ou à un ensemble formé d'un bloc de translation (b1, b2) et un bloc de couplage (c1, c2) qui commandent le déplacement de la surface de préhension (3) ou de l'organe d'application (10), et **en ce que** l'organe de récupération d'énergie (96) comprend un générateur de conversion d'énergie mécanique en énergie électrique (44) tel qu'un générateur électrique tel qu'une dynamo, avec ou sans multiplicateur, associé aux moyens de transmission de couple et comprend un accumulateur d'énergie électrique (45) tel qu'au moins un (super)condensateur et/ou une batterie configurée pour stocker l'énergie électrique ainsi générée par le générateur (44) et la mettre à disposition pour alimenter les moyens d'entraînement (18 ; 51, 60) et/ou le contrôleur électronique (17).

8. Dispositif selon l'une quelconque des revendications précédentes, **caractérisée en ce que** les moyens d'alimentation en liquide (12) comprennent au moins une pompe (13) actionnée de manière contrôlée électroniquement par une unité électronique (17) ou mécaniquement par des moyens d'actionnement mécanique (63, 70, 71, 72) lors de l'actionnement de la poignée de préférence le pivotement de celle-ci autour de son axe d'actionnement (J) pour l'actionnement d'un mécanisme d'ouverture/fermeture de l'ouvrant; ladite pompe étant de préférence une pompe péristaltique ou une pompe à piston.

9. Dispositif selon l'une quelconque des revendications précédentes, **caractérisée en ce que** le réservoir (11) des moyens d'alimentation en liquide (12) est situé au-dessus de la poignée (2), l'apport d'un volume de liquide dispensé à la réserve tampon se faisant par l'ouverture d'une vanne actionnée mécaniquement ou électriquement pour laisser le liquide passer uniquement par la pression due à la force gravitationnelle.

10. Dispositif selon l'une quelconque des revendications précédentes, **caractérisée en ce que** la réserve tampon comprend un tampon applicateur (25) en contact avec la surface de préhension (3) monté de manière démontable dans un support rigide (49) de la bague afin de pouvoir remplacer ou nettoyer le tampon applicateur (25).

11. Dispositif selon la revendication précédente, **caractérisée en ce que** le tampon applicateur est sous forme d'un anneau fendu (26) flexible de sorte à pouvoir passer la poignée à travers une fente (26a) ouverte de l'anneau fendu.

12. Dispositif selon la revendication 10 ou 11, **caractérisée en ce que** le support rigide de la bague comprend au moins deux parties (49a, 49b) séparables, les deux parties verrouillées ensemble par un mécanisme de fixation (152, 154) élastique ou à aimant, et déverrouillable avec un outil (150) comprenant une goupille (151), ou **en ce que** le support rigide comprend au moins deux parties (49a, 49b) séparables, les deux parties verrouillées ensemble par un mécanisme de fixation magnétique (139b).

13. Dispositif selon la revendication précédente, **caractérisée en ce que** les deux parties (49a, 49b) du support rigide de la bague, comprennent une base (49a) et un couvercle (49b), le couvercle étant logé dans la base sous un recouvrement annulaire (49d).

14. Dispositif selon la revendication 12, comprenant en outre un outil de déverrouillage comprenant un aimant produisant un champ magnétique plus fort que le champ magnétique du mécanisme de fixation.

15. Dispositif selon l'une quelconque des revendications précédentes, **caractérisée en ce que** la bague annulaire (49) de l'organe d'application (10) comprend au moins un aimant permanent (139b) logé dans un support rigide (49, 49a, 49b) de la bague, le support rigide comprenant ou constitué d'un matériau ferromagnétique.

16. Dispositif selon l'une quelconque des revendications précédentes, **caractérisée en ce que** la surface de préhension (3) comprend une rainure (54a) circulaire intersectant des trous de distribution (54) des moyens d'alimentation (12) pour l'irrigation d'une zone tampon correspondant à une position initiale de la réserve tampon.

## Patentansprüche

1. Vorrichtung zum Reinigen eines Griffs (1), umfassend mindestens einen Griff (2), der eine zu reinigende Greiffläche (3) umfasst, die Teil einer röhrenförmigen oder teilweise röhrenförmigen Wand ist, ein Auftragselement (10) zum Auftragen einer Reinigungsflüssigkeit auf die Greiffläche (3) und Versorgungsmittel (12) mit Reinigungsflüssigkeit, die mindestens einen Behälter (11) zur Aufnahme und Abgabe von Reinigungsflüssigkeit an das Auftragselement umfassen, wobei das Auftragselement (10) mindestens teilweise aus einem ringförmigen Ring gebildet ist, der um die Greiffläche (3) herum angebracht ist und mindestens eine Pufferreserve (24) umfasst, die in Kontakt mit der Greiffläche (3) eingerichtet ist, wobei die Vorrichtung ferner eine Betätigungseinheit (16) für das Auftragselement umfasst, die Antriebsmittel umfasst, die ausgelegt sind, um das Auftragselement (10) axial entlang der Greiffläche (3) zwischen einer Abgabeposition, in der sich das Auftragselement gegenüber dem Abgabeverteiler befindet, oder in Fluidkommunikation mit diesem, zum Überführen von Reinigungsflüssigkeit in die Pufferreserve (24), und einer Auftragskonfiguration zum Auftragen von Reinigungsflüssigkeit auf die Greiffläche (3) derart zu bewegen, dass bei dieser Bewegung Reinigungsflüssigkeit aus der Pufferreserve (24) auf die Greiffläche aufgetragen wird, wobei die Pufferreserve (24) ein Applikatorkissen (25) in Kontakt mit der Greiffläche (3) umfasst, das eine Struktur zum Speichern von Flüssigkeit in einer porösen oder Zwischenraumstruktur zum Zurückhalten der Flüssigkeit aufweist, so dass die Reinigungsvorrichtung einen Abgabeverteiler (27) für Reinigungsflüssigkeit umfasst, der mindestens einen Flüssigkeitseinlass in Kommunikation mit dem Flüssigkeitsreservoir und eine Vielzahl von Flüssigkeitsauslässen (54) umfasst, die dem Auftragskissen (24) verteilt zugewandt sind.

2. Vorrichtung nach Anspruch 1, **dadurch gekennzeichnet, dass** die Antriebsmittel ausgelegt ist, um das Auftragselement (10) durch magnetische Kopplung zu bewegen.

3. Vorrichtung nach Anspruch 2, **dadurch gekennzeichnet, dass** die Antriebsmittel ein System aus Riemenscheiben (131, 132) und Riemen (133) im Griff umfassen, das ein Verbindungselement (134) umfasst, das fest mit einem Magneten verbunden ist.

4. Vorrichtung nach Anspruch 2, **dadurch gekennzeichnet, dass** die Antriebsmittel eine Schnecke, die sich in Längsrichtung im Inneren der röhrenförmigen Wand der Greiffläche des Griffs erstreckt, und eine Mutter umfassen, die entlang der Schnecke verlagerbar ist, wobei die Mutter einen Magneten (139) antreibt, der den Ring des Reinigungsorgans antreibt, wobei dieser einen magnetischen oder ferromagnetischen Körper (49a, 49b, 139b) umfasst.

5. Vorrichtung nach einem der vorhergehenden Ansprüche, **dadurch gekennzeichnet, dass** die Betätigungseinheit (16) eine elektronische Steuerung (17) umfasst, die ausgelegt ist, um automatisch die Aktion der Antriebsmittel (18, 32) für die Relativbewegung der Pufferreserve (24) mit der Greiffläche (3) zu steuern und/oder die Flüssigkeitsverteilung durch die Versorgungsmittel (12) zu steuern, um die Pufferreserve des Auftragsorgans aus dem Flüssigkeitsreservoir (12) mit Flüssigkeit zu füllen.

6. Vorrichtung nach vorhergehendem Anspruch, **dadurch gekennzeichnet, dass** sie mindestens einen Sensor (15) umfasst, der eingerichtet ist, um das Vorhandensein der Berührung des Griffs durch einen Benutzer zu erfassen, wobei der Sensor der elektronischen Steuerung (17) zugeordnet ist, die ausgelegt ist, um als Reaktion auf eine Erfassungsinformation die Versorgungsmittel (12) mit Flüssigkeit und/oder Antriebsmittel (18, 32) automatisch zu betätigen.

7. Vorrichtung nach einem der vorhergehenden Ansprüche, **dadurch gekennzeichnet, dass** sie mindestens einen Speicher- und Umkehrblock (a1-a4) oder ein Organ zur Rückgewinnung mechanischer Energie (96, 60) durch Mittel zur Drehmomentübertragung (63, 104, 140-144) aufgrund der Drehung des Griffs auf einem Mechanismus zum Öffnen und/oder Schließen eines Flügels (9), zur Speicherung dieser mechanischen Energie und/oder zur Umwandlung und Speicherung als elektrische Energie (45) und schließlich zur Umverteilung dieser gespeicherten mechanischen und/oder elektrischen Energie an die Betätigungseinheit (16) oder an die Antriebsmittel (18, 32; 51, 60) oder an eine aus einem Translationsblock (b1, b2) und einem Kopplungsblock (c1, c2) gebildete Einheit, die die Bewegung der Greiffläche (3) oder des Auftragsorgans (10) steuern, umfasst und dass das Organ zur Energierückgewinnung (96) einen Generator zur Umwandlung von mechanischer Energie in elektrische Energie (44) wie einen elektrischen Generator wie einen Dynamo umfasst, mit oder ohne Getriebe, der den Drehmomentübertragungsmitteln zugeordnet ist und einen elektrischen Energiespeicher (45) wie mindestens einen (Super-)Kondensator und/oder eine Batterie umfasst, die ausgelegt ist, um die derart vom Generator (44) erzeugte elektrische Energie zu speichern und sie zur Versorgung der Antriebsmittel (18; 51, 60) und/oder der elektronischen Steuerung (17) Verfügung zu stellen.

8. Vorrichtung nach einem der vorhergehenden Ansprüche, **dadurch gekennzeichnet, dass** die Versorgungsmittel mit Flüssigkeit (12) mindestens eine Pumpe (13) umfassen, die von einer elektronischen Einheit (17) elektronisch oder von mechanischen Betätigungsmitteln (63, 70, 71, 72) mechanisch bei der Betätigung des Griffs vorzugsweise dem Schwenken desselben um seine Betätigungsachse (J) zur Betätigung eines Mechanismus zum Öffnen/Schließen des Flügels gesteuert betätigt wird; wobei die Pumpe vorzugsweise eine peristaltische Pumpe oder eine Kolbenpumpe ist.

9. Vorrichtung nach einem der vorhergehenden Ansprüche, **dadurch gekennzeichnet, dass** sich das Reservoir (11) der Versorgungsmittel mit Flüssigkeit (12) oberhalb des Griffs (2) befindet, wobei die Zufuhr eines Volumens an gespendeter Flüssigkeit in die Pufferreserve durch das Öffnen eines mechanisch oder elektrisch betätigten Ventils erfolgt, um die Flüssigkeit nur durch den Druck aufgrund der Gravitationskraft passieren zu lassen.

10. Vorrichtung nach einem der vorhergehenden Ansprüche, **dadurch gekennzeichnet, dass** die Pufferreserve ein Applikatorkissen (25) umfasst, das mit der Greiffläche (3) in Kontakt steht und abnehmbar in einem starren Halter (49) des Rings angebracht ist, um das Applikatorkissen (25) austauschen oder reinigen zu können.

11. Vorrichtung nach vorhergehendem Anspruch, **dadurch gekennzeichnet, dass** das Applikatorkissen in Form eines flexiblen Spaltrings (26) vorliegt, so dass der Griff durch einen offenen Schlitz (26a) des Spaltrings hindurchgeführt werden kann.

12. Vorrichtung nach Anspruch 10 oder 11, **dadurch gekennzeichnet, dass** der starre Halter des Rings mindestens zwei trennbare Teile (49a, 49b) umfasst, wobei die beiden Teile durch einen elastischen oder magnetischen Befestigungsmechanismus (152, 154) miteinander verriegelt sind und mit einem Werkzeug (150), das einen Stift (151) umfasst, entriegelbar sind, oder dass der starre Halter mindestens zwei trennbare Teile (49a, 49b) umfasst, wobei die beiden Teile durch einen magnetischen Befestigungsmechanismus (139b) miteinander verriegelt sind.

13. Vorrichtung nach vorhergehendem Anspruch, **dadurch gekennzeichnet, dass** die beiden Teile (49a, 49b) des starren Halters des Rings eine Basis (49a) und einen Deckel (49b) umfassen, wobei der Deckel in der Basis unter einer ringförmigen Abdeckung (49d) untergebracht ist.

14. Vorrichtung nach Anspruch 12, die ferner ein Entriegelungswerkzeug umfasst, das einen Magneten umfasst, der ein Magnetfeld erzeugt, das stärker ist als das Magnetfeld des Befestigungsmechanismus.

15. Vorrichtung nach einem der vorhergehenden Ansprüche, **dadurch gekennzeichnet, dass** der ringförmige Ring (49) des Auftragsorgans (10) mindestens einen Permanentmagneten (139b) umfasst, der in einem starren Halter (49, 49a, 49b) des Rings untergebracht ist, wobei der starre Halter ein ferromagnetisches Material umfasst oder aus diesem besteht.

16. Vorrichtung nach einem der vorhergehenden Ansprüche, **dadurch gekennzeichnet, dass** die Greiffläche (3) eine kreisförmige Nut (54a) umfasst, die Verteilungslöcher (54) der Versorgungsmittel (12) zur Befeuchtung einer Pufferzone schneidet, was einer Anfangsposition der Pufferreserve entspricht.

## Claims

1. A device for cleaning a handle (1) comprising at least one handle (2) comprising a gripping surface (3) to be cleaned forming part of a wall of tubular or partially tubular shape, an application member (10) to apply a cleaning liquid on the gripping surface (3), and cleaning liquid supply means (12) comprising at least one reservoir (11) to receive and distribute cleaning liquid to the application member, the application member (10) formed at least partly of an annular ring mounted around the gripping surface (3) and comprising at least one buffer reserve (24) arranged in contact with the gripping surface (3), the device further comprising a unit (16) for actuating the application member comprising drive means configured to move the application member (10) axially along said gripping surface (3) between a distribution position, in which the application member is opposite the distribution manifold or in fluid communication therewith, for the transfer of cleaning liquid into the buffer reserve (24) and an application configuration for the application of cleaning liquid on the gripping surface (3) so as to apply cleaning liquid from the buffer reserve (24) on the gripping surface during this displacement, the buffer reserve (24) comprising an applicator pad (25) in contact with the gripping surface (3) having a structure for storing the liquid in a porous structure or a structure of gaps for retaining liquid, such that the cleaning device comprises a manifold (27) for distributing cleaning liquid comprising at least one liquid inlet in communication with the liquid reservoir and a plurality of liquid outlets (54) distributed facing the applicator pad (24).

2. The device according to claim 1, **characterized in that** the drive means are configured to move the application member (10) by magnetic coupling.

3. The device according to claim 2, **characterized in that** the drive means comprise a pulley system (131, 132) and a belt (133) in the handle comprising a connection element (134) secured to a magnet.

4. The device according to claim 2, **characterized in that** the drive means comprise a worm screw extending longitudinally inside the tubular wall of the gripping surface of the handle and a nut movable along the worm screw, the nut driving a magnet (139) which drives the ring of the cleaning member, the ring comprising a magnetized or ferromagnetic body (49a, 49b, 139b).

5. The device according to any one of the preceding claims, **characterized in that** the actuation unit (16) comprises an electronic controller (17) configured to automatically control the action of the drive means (18, 32) for the relative displacement of the buffer reserve (24) with the gripping surface (3) and/or control the distribution of liquid by the supply means (12) to fill the buffer reserve of the application member with liquid from the liquid reservoir (12) .

6. The device according to the preceding claim, **characterized in that** it comprises at least one sensor (15) arranged to detect the presence of the touch of the handle by a user, said sensor being associated with the electronic controller (17) which is configured to automatically actuate the liquid supply means (12) and/or drive means (18, 32), in response to detection information.

7. The device according to any one of the preceding claims, **characterized in that** it comprises at least one block (a1-a4) for storing and inverting or one member (96, 60) for recovering a mechanical energy by torque transmission means (63, 104, 140-144) due to the rotation of the handle on a mechanism for opening and/or closing a door leaf (9), for the storage of this mechanical energy and/or for transforming and storing (45) in electrical energy and finally for redistributing this stored mechanical and/or electrical energy to the actuation unit (16) or to the drive means (18, 32; 51, 60) or to an assembly formed of a translation block (b1, b2) and a coupling block (c1, c2) which control the displacement of the gripping surface (3) or of the application member (10), and **in that** the energy recovery member (96) comprises a generator (44) for converting mechanical energy into electrical energy such as an electric generator like a dynamo, with or without a multiplier, associated with the torque transmission means and comprises an electrical energy accumulator (45) such as at least one (super)capacitor and/or one battery configured to store the electrical energy thus generated by the generator (44) and make it available to supply the drive means (18; 51, 60) and/or the electronic controller (17).

8. The device according to any one of the preceding claims, **characterized in that** the liquid supply means (12) comprise at least one pump (13) actuated in a manner controlled electronically by an electronic unit (17) or mechanically by mechanical actuation means (63, 70, 71, 72) during the actuation of the handle preferably the pivoting of the latter about its actuation axis (J) for the actuation of a mechanism for opening/closing the door leaf; said pump being preferably a peristaltic pump or a piston pump.

9. The device according to any one of the preceding claims, **characterized in that** the reservoir (11) of the liquid supply means (12) is located above the handle (2), the supply of a volume of liquid dispensed to the buffer reserve being done by the aperture of a valve actuated mechanically or electrically to let the liquid pass only by the pressure due to the gravitational force.

10. The device according to any one of the preceding claims, **characterized in that** the buffer reserve comprises an applicator pad (25) in contact with the gripping surface (3) mounted in a dismountable manner in a rigid support (49) of the ring in order to be able to replace or clean the applicator pad (25).

11. The device according to the preceding claim, **characterized in that** the applicator pad is in the form of a flexible split annulus (26) so as to be able to pass the handle through an open slot (26a) of the split annulus.

12. The device according to claim 10 or 11, **characterized in that** the rigid support of the ring comprises at least two separable parts (49a, 49b), the two parts locked together by an elastic or magnet fixing mechanism (152, 154), and unlockable with a tool (150) comprising a pin (151), or **in that** the rigid support comprises at least two separable parts (49a, 49b), the two parts locked together by a magnetic fixing mechanism (139b).

13. The device according to the preceding claim, **characterized in that** the two parts (49a, 49b) of the rigid support of the ring, comprise a base (49a) and a cover (49b), the cover being housed in the base under an annular covering (49d) .

14. The device according to claim 12, further comprising an unlocking tool comprising a magnet producing a magnetic field stronger than the magnetic field of the fixing mechanism.

15. The device according to any one of the preceding claims, **characterized in that** the annular ring (49) of the application member (10) comprises at least one permanent magnet (139b) housed in a rigid support (49, 49a, 49b) of the ring, the rigid support comprising or consisting of a ferromagnetic material.

16. The device according to any one of the preceding claims, **characterized in that** the gripping surface (3) comprises a circular groove (54a) intersecting distribution holes (54) of the supply means (12) for the irrigation of a buffer area corresponding to an initial position of the buffer reserve.
